# EUROPEAN PATENT APPLICATION

(11) **EP 1 985 612 A1**
(43) Date of publication of application: **29.10.2008**
(21) Application number: 07090087.3
(22) Date of filing: 26.04.2007
(51) Int. Cl.: C07D 209/16, C07D 215/52, C07D 401/12, C07D 405/12, C07D 409/12, C07C 235/60, A61K 31/404, A61P 5/08

(54) **Arymethylen substituted N-Acyl-gamma-aminoalcohols**

(71) Applicant: Bayer Schering Pharma Aktiengesellschaft, 13353 Berlin (DE)
(72) Inventor: Wortmann, Lars, 10435 Berlin (DE); Menzenbach, Bernd, 07743 Jena (DE); Kosemund, Dirk, 99091 Erfurt (DE); Frenzel, Thomas, 65719 Hofheim (Taunus) (DE); Schrey, Anna Katharina, 10179 Berlin (DE); Kühne, Ronald, 12683 Berlin (DE); Muhn, Peter, 13465 Berlin (DE); Liesener, Florian Peter, 83308 Trostberg (DE); Koppitz, Marcus, 10115 Berlin (DE)

(57) **Abstract**

The present invention relates to arylmethylen substituted N-acyl-γ-aminoalcohols of the general formula I 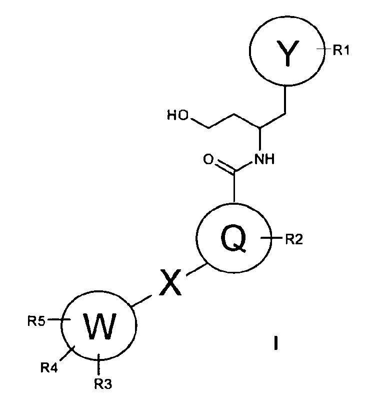 in which Q, X, W, R1, R2, R3, R4 and R5 have the meaning as defined in the description.

The compounds according to the invention are effective FSH modulators and can be used for example for fertility regulation in men or in women.

## Description

The present invention relates to novel arylmethylen substituted N-acyl-γ-aminoalcohols, process for their preparation, pharmaceutical compositions comprising the compounds according to the invention, and the use thereof for fertility regulation in men or women.

Follicle-stimulating hormone (FSH) and luteinizing hormone (LH) are together responsible for the control of male and female fertility and of the production of sex steroids.

In the female mammal, FSH controls the early ripening of ovarian primary follicles and the biosynthesis of sex steroids. In the advanced stage of differentiation (preantral follicles), the influence of LH becomes increasingly important for further development of the follicles until ovulation occurs.

In male mammals, FSH is primarily responsible for the differentiation and stimulation of Sertoli cells. Their function consists of assisting spermatogenesis on many levels. LH is primarily responsible for stimulating the Leydig cells and thus androgen production. FSH, LH and TSH (thyrotropic hormone) together form the group of glycoprotein hormones which are formed in the pituitary and are secreted from there. Whereas the alpha subunit is common to the three hormones, their specificity of action is determined by the beta chain which is unique in each case. The molecular weight of FSH including the sugar portion is about 30 kD.

FSH and the other glycoprotein hormones act specifically via their selectively expressed G protein-coupled receptor (GPCR). FSH stimulates, through binding to its receptor, the association thereof with a stimulating G protein (Gₛ) which is thereby stimulated to hydrolyse guanosine triphosphate (GTP) and to activate the membrane-associated adenylate cyclase. Cyclic adenosine monophosphate (cAMP) is accordingly an important and readily quantifiable secondary messenger substance of FSH (G. Vassart, L. Pardo, S. Costagliola, Trends Biochem. Sci. 2004, 29, 119-126).

The importance of FSH for male fertility is the subject of intensive research. It has been possible to show that FSH influences several processes of spermatogenesis such as the proliferation of spermatogonia, the antiapoptotic effect on spermatogonia and spermatocytes and the stimulation of sperm maturation including motility thereof.

The following arguments are also in favour of the FSH receptor as target for male fertility control:
1. The FSH receptor is exclusively expressed on Sertoli cells (high specificity).
2. Contraceptive vaccination against FSH beta chain or the FSH receptor induces infertility in male primates (N. R. Mougdal, M. Jeyakumar, H. N. Krishnamurthy, S. Sridhar, H. Krishnamurthy, F. Martin, Human Reproduction Update 1997, 3, 335-346).
3. Naturally occurring mutations in the FSH receptor or the FSH beta chain may lead to sub- or infertility in men (I. Huhtaniemi, Journal of Reproduction and Fertility 2000, 119, 173-186; L. C. Layman, P. G. McDonough, Molecular and Cellular Endocrinology 2000, 161, 9-17).
4. Neutralizing FSH antiserum has no effect on testis weight and testosterone production (V. Sriraman, A. J. Rao, Molecular and Cellular Endocrionology 2004, 224, 73-82). Adverse effects of FSH blockade on androgen production therefore appear unlikely.

In line with these arguments, FSH antagonists are expected to be suitable for spermatogenesis inhibition (prevention) in men. Moreover, a suitable FSH antagonist may just as well lead to infertility in women, because it suppresses follicle ripening and thus also ovulation. On the other hand, the skilled person expects advantages from non-peptidergic FSH agonists when used to promote fertility in women (stimulation of follicle ripening). There are no reports of experience on the use of FSH or FSH agonists in male infertility, but specific indications are also conceivable in this connection.

New findings demonstrate that there is also a direct effect of FSH on cells of bone metabolism. There are two fundamentally different cell types in bones: osteoclasts and osteoblasts. While osteoclasts play a central role in bone resorption (breakdown of bone), osteoblasts simulate bone density (anabolic effect).

FSH receptors have been detected in osteoclasts but not in osteoblasts. In vitro, FSH stimulates bone resorption by mouse osteoclasts ( Li Sun et al. Cell 2006; 125: 247-60). A clinical correlation between the height of the serum FSH levels and low bone density has been observed in postmenopausal women (Devleta et al, J. Bone Miner. Metab. 2004, 22: 360-4).

These findings among others suggest that FSH stimulates loss of bone mass, and accordingly FSH antagonists will display an antiresorptive effect on bone and are therefore suitable for the therapy and/or prevention of peri- and postmenopausal loss of bone mass and osteoporosis.

FSH receptor agonists are disclosed in WO 02/09706; J. Comb. Chem. 2004, 6, 196 [Thiazolidinones]; WO 2003/020726 and WO 03/20727, Chem. Biochem. 2002, 10, 1023 {thieno[2,3-d] pyrimidines)}; WO 01/87287 [pyrazoles]; WO 00/08015 [carbazoles]; WO 06/117023, WO 06/117368, WO 06/117370, WO 06/117371, [hexahydroquino-lines].

FSH receptor antagonists are disclosed in WO 03/004028 [tetrahydroquinolines], WO 02/09705 [thiazolidinones], WO 00/58277, Bioorg. Med. Chem. 2002, 10, 639 [sulphonic acids]; WO 00/58276, Endocr. 2002, 143, 3822; Synth. Comm. 2002, 32, 2695 [azo compounds]; US 2006/0199806, US 2006/0258644, US 2006/0258645, US 2006/0287522 [pyrrolobenzodiazepines], WO 2007/017289 [acyltryptophanols], EP 06090223.6 [1,2-diarylacetylene derivatives of acyltryptophanols], EP 06077263.9 [bicyclic acyltryptophanols], EP 07090034.5 [sulfonyltryptophanols] and EP 07090059.2 [tetrahydrocarbazoles].

FSH receptor modulators are compounds that selectively have modulatory activity on the FSH receptor and can be either used as (partial) agonists or (partial) antagonists of the FSH receptor (WO 2004/056780, WO 2004/056779).

Further FSH receptor modulators of various compound classes of low molecular weight were described in J. Med. Chem. 2005, 48, 1697 [tetrahydroquinolines]; WO 02/70493, Bioorg. Med. Chem. Lett. 2004, 14, 1713 and 1717 [diketopiperazines]; and WO 01/47875 [sulphonamides].

WO 2007/017289, EP06090223.6 [1,2-diarylacetylene derivatives of acyltryptophanols] and EP06077263.9 [bicyclic acyltryptophanols], EP 07090059.2 [tetrahydrocarbazoles] disclose compounds with FSH receptor antagonistic activity that come structurally closest to the compounds of the present invention. Compounds according to the present invention differ from the acyl tryptophanol derivatives disclosed in WO 2007/017289 in that the aryl/heteroaryl group Y is part of a homoamino alcohol (i.e. homotryptophanol) moiety rather than tryptophanol.

The closest prior art is considered to have been disclosed in either WO 2004/056779 or WO 2004/056780, i.e. compounds with a FSH receptor modulator activity profile. Compounds according to the present invention are structurally different from those of the closest prior art.

WO 03/035076 discloses dihydroxypyrimidine carboxamides, which are inhibitors of the HIV integrase. Compounds disclosed therein either specifically or generically do not fall under the scope the present invention because the dihydropyridine moiety of compounds WO 03/035076 invariable bears at least one hydroxy group.

Neither does formula I of the present invention overlap with a sub-scope of compounds disclosed generically in WO 2005/087700, claimed to be vitamin D3 receptor agonists, because of X not being a C1-group.

Due to the provision for the groups R2 and (R3)(R4)(R5)W if Q is phenyl or pyridyl, nor does the scope of formula I according to the present invention overlap with the scope of compounds disclosed generically in WO 2005/085227, which claims AKT kinase inhibitors as anticancer agents.

In view of the prior art, the objective technical problem to be solved according to the present invention may therefore be seen in providing alternative compounds having an FSH receptor modulatory effect.

The technical problem has been solved according to the present invention by the provision of novel compounds of the formula I 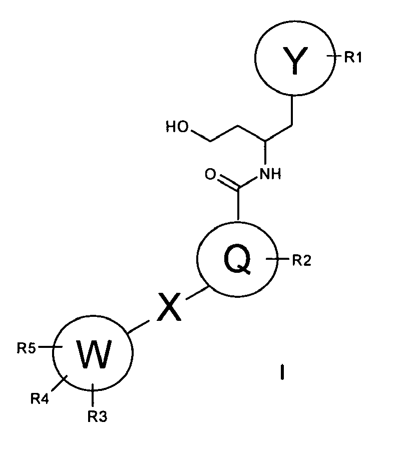 in which
- R1: is hydrogen, halogen, cyano, C₁-C₆-alkyl;
- R2: is hydrogen, halogen, cyano, C₁-C₆-alkyl, C₂-C₆-alkenyl or C₂-C₆-alkynyl, C₃-C₇-cycloalkyl, C₃-C₇-heterocycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene where the hydrocarbon chains therein may optionally be substituted one or more times by fluorine or cyano;
- R3, R4, R5: are independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or the radicals: 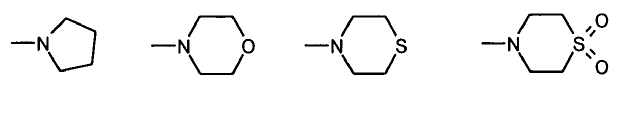 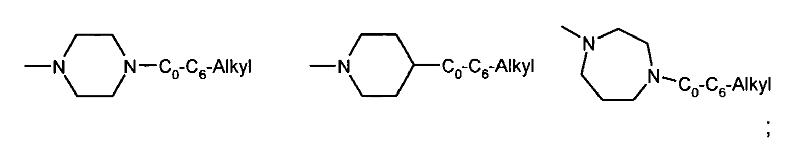
or
- R3, R4, R5: are independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneamino-carbonyl,
C₁-C₆-alkylcarbonyl, C₃-C₇-cycloalkylcarbonyl, carboxy, carboxamido [-C(O)NH₂], C₁-C₆-alkyloxycarbonyl,
C₁-C₃-alkylsulphanyl, C₁-C₆-alkysulphonyl, C₃-C₇-cycloalkylsulphonyl,
C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido,
-N(C₀-C₆-alkyl)-C(O)-C₁-C₆-alkyl, -N(C₀-C₆-alkyl)-C(O)-C₃-C₇-cycloalkyl,
-N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂-C₃-C₇-cycloalkyl,
-N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the radicals: 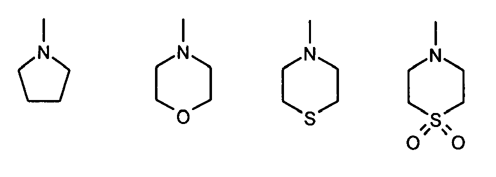 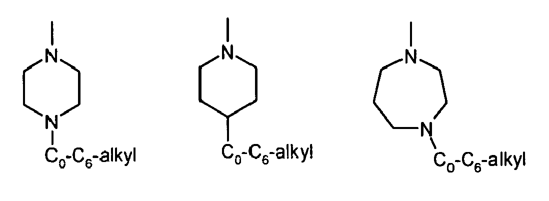 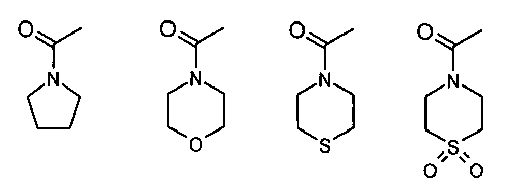 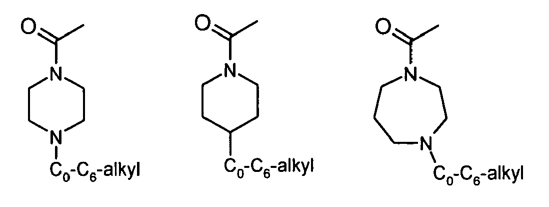 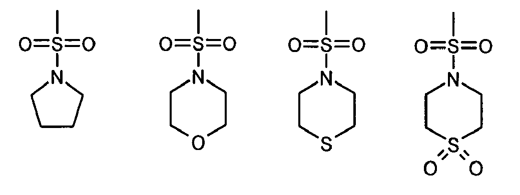 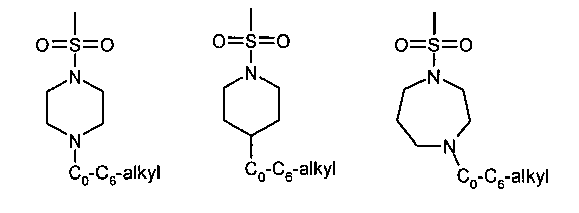 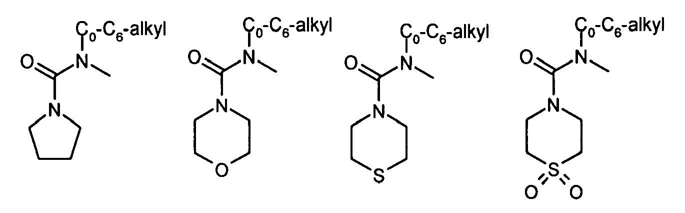 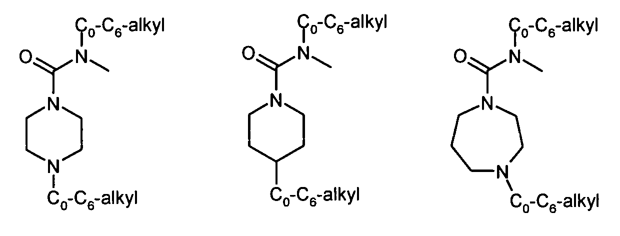 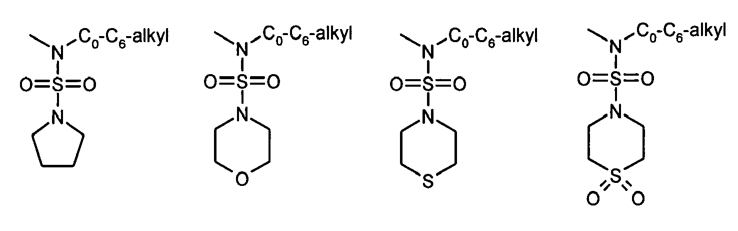 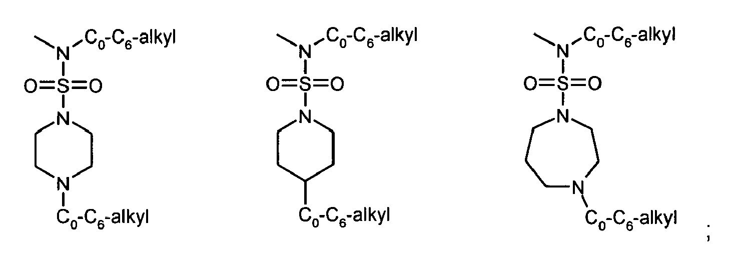
- R3 and R4: may together form heterocycloalkyl, cycloalkyl;
- Q: is an aryl or heteroaryl group
or the biradical group 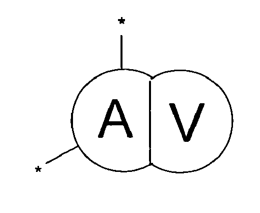 in which
A is a monocyclic aryl or a monocyclic heteroaryl group;
V is a cycloalkylen, cycloalkenylen, heterocycloalkylen or heterocycloalkenylen group;
- X: is a bond or ethynylene;
- W: is an aryl or heteroaryl group;
- Y: is an aryl or heteroaryl group;
where
- R1: substitutes one or more positions of the aryl or heteroaryl ring in the radical Y;
- R2: substitutes one or more positions of the aryl or heteroaryl ring in the radical V, or
- R2: substitutes one or more positions of the aryl or heteroaryl ring in the radical Q
with the provision that
if Q is a phenyl or pyridyl group
then
1) the R2 substituent in the para-position relative to the amide group may only be hydrogen; and
2) the group 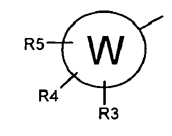
may not be a benzamide, substituted benzamide, phenylethanon, substituted phenylethanon, furan or thiophene.

The present invention relates to both possible enantiomeric forms at the stereocentre of the aminoalcohol residue.

The unbranched C₁-C₆-alkyl groups for the radicals R1 to R5 may be for example a methyl, ethyl, propyl, butyl, pentyl or a hexyl group; and the branched C₃-C₆-alkyl groups for the radicals R1 to R6 may be an isopropyl, isobutyl, sec-butyl, *tert*-butyl, isopentyl, 2-methylbutyl, 1-methylbutyl, 1-ethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or a 1,2-dimethylbutyl group.

The branched or unbranched C₂-C₆-alkenyl groups for the radicals R2 to R5 may be for example a vinyl, allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethyl-prop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methyl-pent-3-enyl, (*Z*)-1-methylpent-3-enyl, (*E*)-4-methylpent-2-enyl,(Z)-4-methylpent-2-enyl, (*E*)-3-methylpent-2-enyl, (*Z*)-3-methylpent-2-enyl, (*E*)-2-methylpent-2-enyl, (*Z*)-2-methyl-pent-2-enyl, (*E*)-1-methylpent-2-enyl,(*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methyl-pent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethyl-but-2-enyl, (*E*)-2-ethylbut-2-enyl, (*Z*)-2-ethylbut-2-enyl, (*E*)-1-ethylbut-2-enyl, (*Z*)-1-ethyl-but-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropyl-prop-1-enyl, (*E*)-1-isopropylprop-1-enyl, (*Z*)-1-isopropylprop-1-enyl, (*E*)-3,3-dimethyl-prop-1-enyl, (*Z*)-3,3-dimethylprop-1-enyl- or a 1-(1,1-dimethylethyl)ethenyl group.

The C₂-C₆-alkynyl groups for the radicals R2 to R5 may be for example an ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.

The C₁-C₆-alkyloxy groups for the radicals R2 to R5 may be for example a methyloxy, ethyloxy, propyloxy, *iso*propyloxy, butyloxy, *iso*butyloxy, *sec*-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy group.

The halogens for the radicals R1 to R5 are fluorine, chlorine, bromine or iodine.

The C₁-C₃-alkylsulphanyl groups for the radicals R3 to R5 may be for example a methylsulphanyl (CH₃S-), ethylsulphanyl (CH₃CH₂S-), propylsulphanyl, *iso*propyl-sulphanyl group.

The C₁-C₆-alkylaminocarbonyl groups for the radicals R3 to R5 may be for example a methylaminocarbonyl-, ethylaminocarbonyl-, propylaminocarbonyl-, isopropylaminocarbonyl-, butylaminocarbonyl-, isobutylaminocarbonyl-, sec-butylaminocarbonyl-, *tert-*butylaminocarbonyl-, pentylaminocarbonyl-, isopentylaminocarbonyl-, (2-methylbutyl)-aminocarbonyl-, (1-methylbutyl)aminocarbonyl-, (1-ethylpropyl)aminocarbonyl-, neo-pentylaminocarbonyl-, (1,1-dimethylpropyl)aminocarbonyl-, hexylaminocarbonyl-, (4-methylpentyl)aminocarbonyl-, (3-methylpentyl)aminocarbonyl-, (2-methylpentyl)aminocarbonyl-, (1-methylpentyl)aminocarbonyl-, (1-ethylbutyl)aminocarbonyl-, (2-ethylbutyl)-aminocarbonyl-, (3,3-dimethylbutyl)aminocarbonyl-, (2,2-dimethylbutyl)aminocarbonyl-, (1,1-dimethylbutyl)aminocarbonyl-, (2,3-dimethylbutyl)aminocarbonyl-, (1,3-dimethylbutyl)aminocarbonyl- or a (1,2-dimethylbutyl)aminocarbonyl group.

The hydroxy-C₁-C₆-alkylene groups for the radicals R2 to R5 may be a hydroxymethyl (HOCH₂-), 2-hydroxyethyl (HOCH₂CH₂-), 1-hydroxyethyl [CH₃CH(OH)-], 3-hydroxypropyl (HOCH₂CH₂CH₂-), 2-hydroxypropyl [CH₃CH(OH)CH₂-], 1-hydroxypropyl [CH₃CH₂CH(OH)-], 2-hydroxy-1-methylethyl [HOCH₂CH(CH₃)-], 1-hydroxy-1-methylethyl [(CH₃)₂C(OH)-], 4-hydroxybutyl (HOCH₂CH₂CH₂CH₂-), 3-hydroxybutyl [CH₃CH(OH)CH₂CH₂-], 2-hydroxybutyl [CH₃CH₂CH(OH)CH₂-], 1-hydroxybutyl [CH₃CH₂CH₂CH(OH)-], 3-hydroxy-1-methylpropyl [HOCH₂CH₂CH(CH₃)-], 2-hydroxy-1-methylpropyl [CH₃CH(OH)CH(CH₃)-], 1-hydroxy-l-methylpropyl [CH₃CH₂C(CH₃)(OH)-], 1-(hydroxymethyl)propyl [CH₃CH(CH₂OH)-], 3-hydroxy-2-methylpropyl [HOCH₂CH(CH₃)CH₂-], 2-hydroxy-2-methylpropyl [(CH₃)₂C(OH)CH₂-], 1-hydroxy-2-methylpropyl [CH₃CH(CH₃)CH(OH)-] or a 2-hydroxy-1,1-dimethylethyl group [HOCH₂C(CH₃)₂-].

The heterocycloalkyl groups which may form the radicals R3 and R4 together may be for example the following groups:

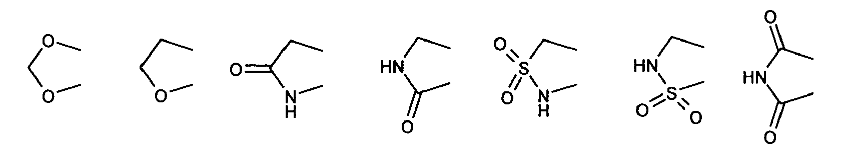

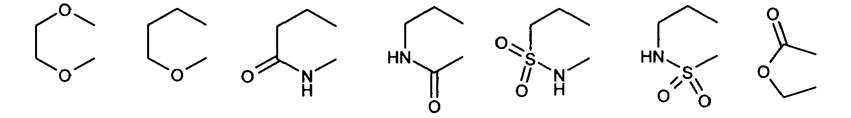

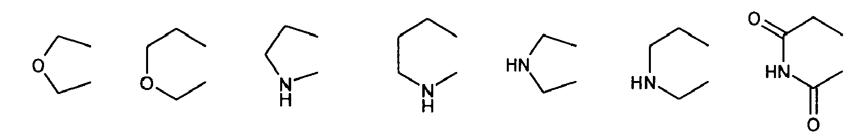

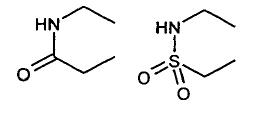

The cycloalkyl groups which may form the radicals R3 and R4 together may be for example the following groups:

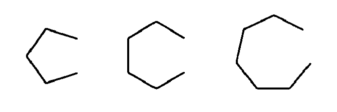

The C₃-C₇-cycloalkyl groups for the radicals R2 to R5 may be for example a cyclopropyl, cyclobutyl, cyclopentyl, cyclohexyl, cycloheptyl group.

The C₃-C₇-heterocycloalkyl groups for the radicals R3 to R5 may be for example a cyclopropyl, cyclobutyl, cycopentyl, cyclohexyl, cycloheptyl group in which one or two carbon atoms of the ring are replaced independently of one another by an oxygen, nitrogen or sulphur atom.

The monocyclic aryl group for A or Y may be for example a phenyl group which is linked via substitutable positions.

The aryl group for W or Q may be for example a phenyl, naphthyl group which is linked via substitutable positions.

The monocylic heteroaryl group for A or Y may be for example a pyridinyl, pyrimidinyl, furanyl, thienyl, oxazolyl, isoxazolyl, thiazolyl, pyrrolyl, pyrazolyl or an imidazolyl group which is linked via substitutable positions.

The heteroaryl group for W or Q may be for example a pyridinyl, pyrimidinyl, quinolinyl, isoquinolinyl, quinazolinyl, quinoxalinyl, phthalazinyl, 1.5-naphthyridinyl, 1,6-naphthyridinyl, 1,7-naphthyridinyl, 1,8-naphthyridinyl, benzofuranyl, benzothienyl, 1,3-benzodioxolyl, 2,1,3-benzothiadiazolyl, indolyl, indazolyl, furanyl, thienyl, oxazolyl, isoxazolyl, thiazolyl, pyrrolyl, pyrazolyl or an imidazolyl group which is linked via substitutable positions.

The heterocycloalkylen groups for V or Z may be for example the following groups:

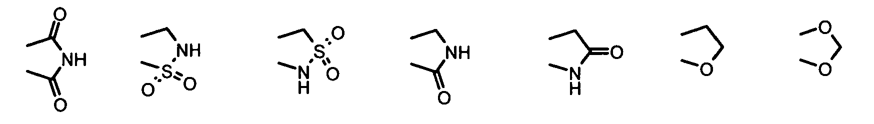

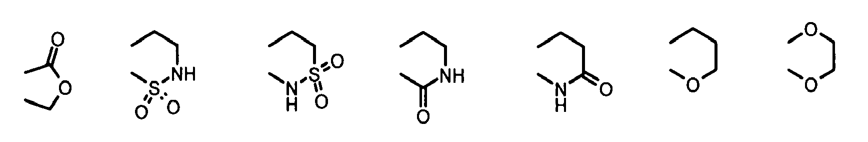

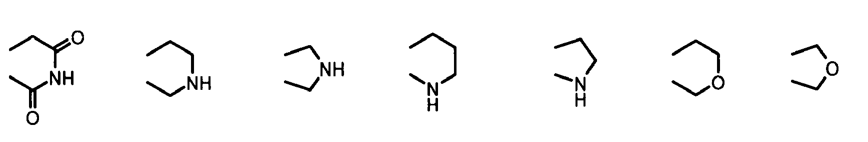

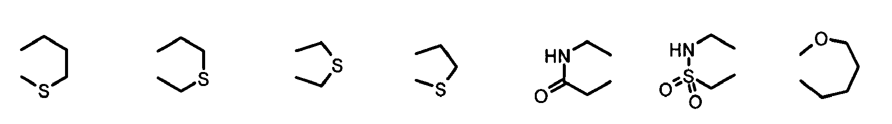

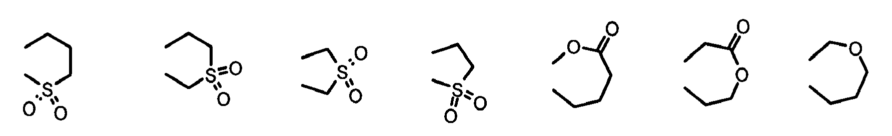

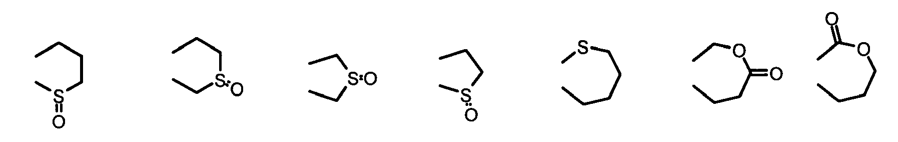

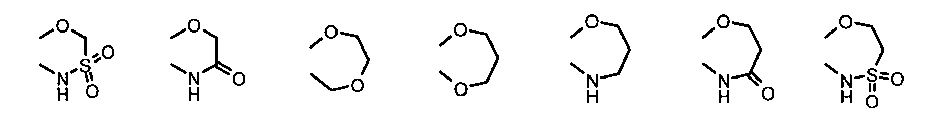

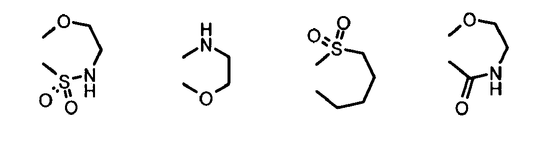

The heterocycloalkenylen groups for V or Z may be for example the following groups:

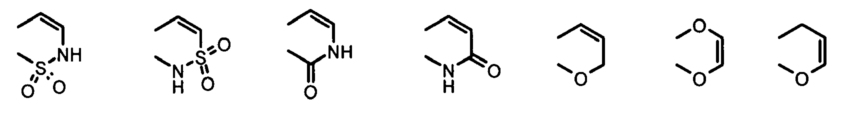

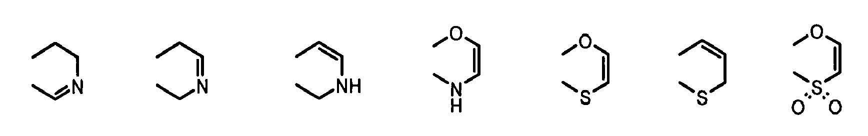

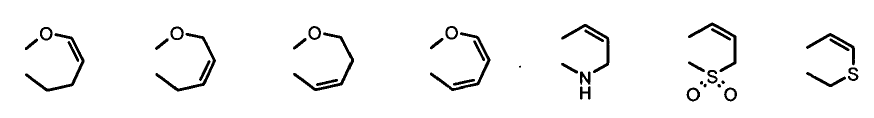

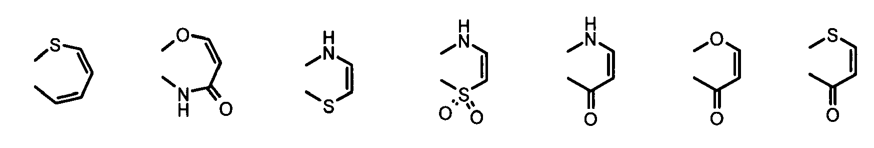

The cycloalkylen groups for V or Z may be for example the following groups:

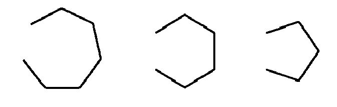

The cycloalkenylen groups for V or Z may be for example the following groups:

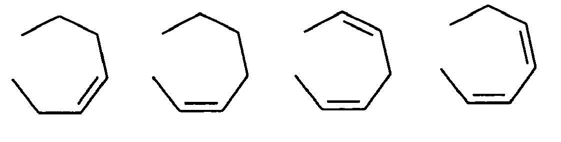

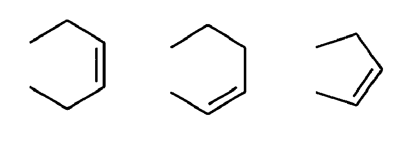

The C₁-C₄-alkylene groups for the radicals X may be for example a methylene (-CH₂-), ethylidene [-CH(CH₃)-], ethylene (-CH₂CH₂-), prop-1,3-ylene (-CH₂CH₂CH₂-), prop-1,2-ylene [-CH₂CH(CH₃)-], but-1,4-ylene (-CH₂CH₂CH₂CH₂-), but-1,3-ylene [-CH₂CH₂CH(CH₃)-], but-1,2-ylene [-CH₂CH(CH₂CH₃)-], but-2,3-ylene [-CHCH(CH₃)-], 2-methylprop-1,2-ylene [-CH₂C(CH₃)₂-] or a 2-methylprop-1,3-ylene group [-CH₂CH(CH₃)CH₂-].

The C₂-C₄-alkenylene groups for the radical X may be for example an ethen-1,2-ylidene (-CH=CH-), prop-2-en-1,3-ylidene (-CH₂-CH=CH-), prop-1-en-1,3-ylidene (-CH=CH-CH₂-), but-1-en-1,4-ylidene (-CH=CH-CH₂-CH₂-), but-2-en-1,4-ylidene (-CH₂-CH=CH-CH₂-) or a but-3-en-1,4-ylidene group (-CH₂-CH₂-CH=CH-).

The C₂-C₄-alkynylene groups for the radical X may be for example an ethyn-1,2-ylidene (-C≡C-), prop-2-yn-1,3-ylidene (-CH₂-C≡C-), prop-1-yn-1,3-ylidene (-C≡C-CH₂-), but-1-yn-1,4-ylidene (-C≡C-CH₂-CH₂-), but-2-yn-1,4-ylidene (-CH₂-C≡C-CH₂-) or a but-3-yn-1,4-ylidene group (-CH₂-CH₂-C≡C-).

The C₃-C₇-cycloalkyloxy groups for the radicals R2 to R5 may be for example a cyclo-propyloxy, cyclobutyloxy, cyclopentyloxy, cyclohexyloxy, cycloheptyloxy group.

The C₁-C₆-alkylamino groups for the radicals R3 to R5 may be for example methyl-amino, ethylamino, propylamino, *iso*propylamino, butylamino, *iso*butylamino, *sec*-butylamino, *tert*-butylamino, pentylamino, *iso*pentylamino, (2-methylbutyl)amino, (1-methylbutyl)amino, (1-ethylpropyl)amino, *neo*pentylamino, (1,1-dimethylpropyl)amino, hexyl-amino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino group.

In the di(C₁-C₆-alkyl)amino groups for the radicals R3 to R5, each of the two radicals on the nitrogen atom of the dialkylamino group may be chosen independently of one another from the following radicals: possible examples are a methyl, ethyl, propyl, isopropyl, butyl, *iso*butyl, *sec*-butyl, *tert*-butyl, pentyl, *iso*pentyl, (2-methylbutyl), (1-methylbutyl), (1-ethylpropyl), *neo*pentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the C₃-C₇-cycloalkyl-C₁-C₆-alkyleneoxy groups for the radicals R2 to R5 it is possible to combine each of the C₃-C₇-cycloalkyl groups of the C₃-C₇-cydoalkyl-C₁-C₆-alkyleneoxy group, for example of a cyclopropyl, cyclobutyl, cyclopentyl, cyclohexyl or cycloheptyl group, independently of one another with each C₀-C₆-alkyleneoxy group, for example with a methyleneoxy, ethyleneoxy, propyleneoxy, butyleneoxy, pentyleneoxy, hexyleneoxy group.

In the hydroxy-C₃-C₆-alkenylene groups for the radicals R2 to R5 it is possible for the hydroxy group to be located on any desired position of the C₃-C₆-alkenyl group, for example of an allyl, (*E*)-2-methylvinyl, (*Z*)-2-methylvinyl, homoallyl, (*E*)-but-2-enyl, (Z)-but-2-enyl, (*E*)-but-l-enyl, (*Z*)-but-1-enyl, pent-4-enyl, (*E*)-pent-3-enyl, (*Z*)-pent-3-enyl, (*E*)-Pent-2-enyl-, (*Z*)-Pent-2-enyl-, (*E*)-Pent-1-enyl-, (*Z*)-Pent-1-enyl-, hex-5-enyl-, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethyl-prop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (Z)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethyl-but-2-enyl, (*E*)-2-ethylbut-2-enyl, (Z)-2-ethylbut-2-enyl, (*E*)-1-ethylbut-2-enyl, (*Z*)-1-ethyl-but-2-enyl, (*E*)-3-ethylbut-l-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isoprop-ylprop-l-enyl, (*E*)-1-isopropylprop-1-enyl, (*Z*)-1-isopropylprop-1-enyl, (*E*)-3,3-dimethyl-prop-1-enyl, (*Z*)-3,3-dimethylprop-1-enyl or a 1-(1,1-dimethylethyl)ethenyl group, and to be combined independently of one another.

In the hydroxy-C₃-C₆-alkynyl groups for the radicals R2 to R5 it is possible for the hydroxy group to be located at any desired position of the C₃-C₆-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-di-methylbut-3-ynyl, 1,1-dimethylbut-3-ynyl, 1,1-dimethylbut-2-ynyl or a 3,3-dimethylbut-l-ynyl group.

In the C₁-C₆-alkyloxy-C₃-C₆-alkenylene groups for the radicals R2 to R5 it is possible for the C₁-C₆-alkyloxy group, for example a methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (Z)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methyl-prop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (Z)-2-methylpent-3-enyl, (*E*)-1-methylpent-3-enyl, (*Z*)-1-methylpent-3-enyl, (*E*)-4-methylpent-2-enyl, (Z)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (Z)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-l-enyl, (*Z*)-1-propylprop-l-enyl, (*E*)-2-isopropylprop-1-enyl, (*Z*)-2-isopropylprop-1-enyl, (*E*)-1-isopropylprop-l-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group and to be combined independently of one another.

In the C₁-C₆-alkyloxy-C₃-C₆-alkynylene groups for the radicals R2 to R5 it is possible for the C₁-C₆-alkyloxy group, for example a methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located at any desired position of the C3-C6-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methyl-but-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-di-methylbut-3-ynyl, 1,1-dimethylbut-3-ynyl, 1,1-dimethylbut-2-ynyl or a 3,3-dimethylbut-1-ynyl group, and to be combined independently of one another.

In the C₁-C₆-alkyloxyphenyl-C₁-C₆-alkylene groups for the radical R3 to R5 it is possible for the C₁-C₆-alkyloxy group to be selected independently of one another from methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy, and to be combined independently of one another with C₁-C₆-alkylene groups such as, for example, methylene, ethylene, propylene, butylene, pentylene, hexylene.

In the C₃-C₇-cycloalkyl-(C₀-C₆)-alkyleneamino groups of the radicals R3 to R5 it is possible for each of the C₃-C₇-cycloalkyl groups of the C₃-C₇-cycloalkyl-(C₀-C₆)-alkylene-amino group, for example of a cyclopropyl, cyclobutyl, cyclopentyl, cyclohexyl or cycloheptyl group, to be combined independently of one another with each C₀-C₆-alkylene group, for example with a bond, a methylene, ethylene, propylene, butylene, pentylene, hexylene group.

In the C₁-C₆-alkyloxy-C₁-C₆-alkylene groups for the radical R2 to R5, it is possible for the C₁-C₆-alkyloxy group to be selected independently for example from methyloxy, ethyloxy, propyloxy, *iso*propyloxy, butyloxy, *iso*butyloxy, *sec*-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy and to be combined independently of one another with C₁-C₆-alkylene groups such as, for example, methylene, ethylene, propylene, butylene, pentylene, hexylene.

In the di(C₁-C₆-alkyl)amino-C₁-C₆-alkylene group for the radicals R2 to R5 it is possible for each of the two radicals on the nitrogen atom of the amino group to be selected independently for example from methyl, ethyl, propyl, isopropyl, butyl, *iso*butyl, *sec*-butyl, *tert*-butyl, pentyl, *iso*pentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, and to be combined with C₁-C₆-alkylene groups such as, for example, methylene, ethylene, propylene, butylene, pentylene, hexylene.

The C₃-C₇-cycloalkyl-C₁-C₆-alkylene groups for the radicals R2 to R5 may be for example a cyclopropyloxymethylene, cyclopropyloxyethylene, cyclopropyloxypropylene, cyclopropyloxybutylene, cyclopropyloxypentylene, cyclopropyloxyhexylene, cyclobutyloxymethylene, cyclobutyloxyethylene, cyclobutyloxypropylene, cyclobutyloxybutylene, cyclobutyloxypentylene, cyclobutyloxyhexylene, cyclopentyloxymethylene, cyclopentyloxyethylene, cyclopentyloxypropylene, cyclopentyloxybutylene, cyclopentyloxypentylene, cyclopentyloxyhexylene, cyclohexyloxymethylene, cyclohexyloxyethylene, cyclohexyloxypropylene, cyclohexyloxybutylene, cyclohexyloxypentylene, cyclohexyloxyhexylene, cycloheptyloxymethylene, cycloheptyloxyethylene, cycloheptyloxypropylene, cycloheptyloxybutylene, cycloheptyloxypentylene, cycloheptyloxyhexylen group.

In the C₁-C₆-alkylamino-C₁-C₆-alkylene groups for the radicals R3 to R5 it is possible for the C₁-C₆-alkylamino group to be selected independently for example from methyl-amino, ethylamino, propylamino, *iso*propylamino, butylamino, *iso*butylamino, *sec*-butylamino, *tert*-butylamino, pentylamino, *iso*pentylamino, (2-methylbutyl)amino, (1-methylbutyl)amino, (1-ethylpropyl)amino, *neopent*ylamino, (1,1-dimethylpropyl)amino, hexyl-amino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino and to be combined with C₁-C₆-alkylene groups such as, for example, methylene, ethylene, propylene, butylene, pentylene, hexylene.

The phenyloxy-C₁-C₆-alkylene groups for the radicals R3 to R5 may be for example a phenyloxymethyl, phenyloxyethyl, phenyloxypropyl, phenyloxybutyl, phenyloxypentyl, phenyloxyhexyl group.

In the C₁-C₆-acyl-(C₀-C₆-alkyl)amido groups for the radicals R3 to R5, it is possible for each of the C₁-C₆-acyl groups, for example a formyl, acetyl, propionyl, 2-methylpropionyl, 2,2-dimethylpropionyl, butyryl, 2-methylbutyryl, 3-methylbutyryl, 2,2-dimethyl-butyryl, 2-ethylbutyryl, pentanoyl, 2-methylpentanoyl, 3-methylpentanoyl, 4-methylpentanoyl or a hexanoyl group, to be combined independently of one another with each (C₀-C₆-alkyl)amido group, for example a hydrogen atom, a methylamido, ethylamido, propylamido, isopropylamido, butylamido, isobutylamido, sec-butylamido, tert-butylamido, pentylamido, isopentylamido, (2-methylbutyl)amido, (1-methylbutyl)amido, (1-ethylpropyl)amido, neopentylamido, (1,1-dimethylpropyl)amido, hexylamido, (4-methylpentyl)amido, (3-methylpentyl)amido, (2-methylpentyl)amido, (1-methylpentyl)amido, (1-ethylbutyl)amido, (2-ethylbutyl)amido, (3,3-dimethylbutyl)amido, (2,2-dimethylbutyl)-amido, (1,1-dimethylbutyl)amido, (2,3-dimethylbutyl)amido, (1,3-dimethylbutyl)amido or a (1,2-dimethylbutyl)amido group.

The C₁-C₆-alkylaminocarbonyl groups for the radicals R3 to R5 may be for example a methylaminocarbonyl, ethylaminocarbonyl, propylaminocarbonyl, isopropylaminocarbonyl, butylaminocarbonyl, isobutylaminocarbonyl, sec-butylaminocarbonyl, tert-butylaminocarbonyl, pentylaminocarbonyl, isopentylaminocarbonyl, (2-methylbutyl)-aminocarbonyl, (1-methylbutyl)aminocarbonyl, (1-ethylpropyl)aminocarbonyl, neopentylaminocarbonyl, (1,1-dimethylpropyl)aminocarbonyl, hexylaminocarbonyl, (4-methylpentyl)aminocarbonyl, (3-methylpentyl)aminocarbonyl, (2-methylpentyl)aminocarbonyl, (1-methylpentyl)aminocarbonyl, (1-ethylbutyl)aminocarbonyl, (2-ethylbutyl)aminocarbonyl, (3,3-dimethylbutyl)aminocarbonyl, (2,2-dimethylbutyl)aminocarbonyl, (1,1-dimethylbutyl)aminocarbonyl, (2,3-dimethylbutyl)aminocarbonyl, (1,3-dimethylbutyl)-aminocarbonyl or a (1,2-dimethylbutyl)aminocarbonyl group.

In the di(C₁-C₆-alkyl)aminocarbonyl groups for the radicals R3 to R5, each of the two C₁-C₆-alkyl radicals on the nitrogen atom of the di(C₁-C₆-alkyl)aminocarbonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

The (C₃-C₇-cycloalkyl)aminocarbonyl groups for the radicals R3 to R5 may be for example a cyclopropylaminocarbonyl, cyclobutylaminocarbonyl, cyclopentylaminocarbonyl, cyclohexylaminocarbonyl or cycloheptylaminocarbonyl group.

In the di(C₃-C₇-cycloalkyl)aminocarbonyl groups for the radicals R3 to R5, each of the two C₃-C₇-cycloalkyl radicals on the nitrogen atom of the di(C₃-C₇-cycloalkyl)aminocarbonyl group may be independently of one another for example a cyclopropyl, cyclobutyl, cyclopentyl, cyclohexyl or cycloheptyl group.

In the C₃-C₇-cycloalkyl-C₁-C₆-alkyleneaminocarbonyl groups of the radicals R3 to R5 it is possible for each of the C₃-C₇-cycloalkyl groups of the C₃-C₇-cycloalkyl-C₁-C₆-alkyleneaminocarbonyl groups, for example of a cyclopropyl, cyclobutyl, cyclopentyl, cyclohexyl or cycloheptyl group, to be combined independently of one another with each C₁-C₆-alkyleneaminocarbonyl group, for example with a methyleneaminocarbonyl, ethyleneaminocarbonyl, propyleneaminocarbonyl, butyleneaminocarbonyl, pentylene-aminocarbonyl, hexyleneaminocarbonyl group.

The C₁-C₆-alkylcarbonyl groups for the radicals R3 to R5 may be for example a methyl-carbonyl, ethylcarbonyl, propylcarbonyl, isopropylcarbonyl, butylcarbonyl, isobutylcarbonyl, sec-butylcarbonyl, tert-butylcarbonyl, pentylcarbonyl, isopentylcarbonyl, (2-methylbutyl)carbonyl, (1-methylbutyl)carbonyl, (1-ethylpropyl)carbonyl, neopentylcarbonyl, (1,1-dimethylpropyl)carbonyl, hexylcarbonyl, (4-methylpentyl)carbonyl, (3-methylpentyl)carbonyl, (2-methylpentyl)carbonyl, (1-methylpentyl)carbonyl, (1-ethylbutyl)-carbonyl, (2-ethylbutyl)carbonyl, (3,3-dimethylbutyl)carbonyl, (2,2-dimethylbutyl)carbonyl, (1,1-dimethylbutyl)carbonyl, (2,3-dimethylbutyl)carbonyl, (1,3-dimethylbutyl)-carbonyl or a (1,2-dimethylbutyl)carbonyl group.

The C₃-C₇-cycloalkylcarbonyl groups for the radicals R3 to R5 may be for example a cyclopropylcarbonyl, cyclobutylcarbonyl, cyclopentylcarbonyl, cyclohexylcarbonyl or cycloheptylcarbonyl group.

The C₁-C₆-alkyloxycarbonyl groups for the radicals R3 to R5 may be for example a methyloxycarbonyl, ethyloxycarbonyl, propyloxycarbonyl, isopropyloxycarbonyl, butyloxycarbonyl, isobutyloxycarbonyl, sec-butyloxycarbonyl, tert-butyloxycarbonyl, pentyloxycarbonyl, isopentyloxycarbonyl, (2-methylbutyl)oxycarbonyl, (1-methylbutyl)oxycarbonyl, (1-ethylpropyl)oxycarbonyl, neopentyloxycarbonyl, (1,1-dimethylpropyl)oxycarbonyl, hexyloxycarbonyl, (4-methylpentyl)oxycarbonyl, (3-methylpentyl)oxycarbonyl, (2-methylpentyl)oxycarbonyl, (1-methylpentyl)oxycarbonyl, (1-ethylbutyl)oxycarbonyl, (2-ethylbutyl)oxycarbonyl, (3,3-dimethylbutyl)oxycarbonyl, (2,2-dimethylbutyl)oxycarbonyl, (1,1-dimethylbutyl)oxycarbonyl, (2,3-dimethylbutyl)oxycarbonyl, (1,3-dimethylbutyl)oxycarbonyl or a (1,2-dimethylbutyl)oxycarbonyl group.

The C₁-C₆-alkylsulphonyl groups for the radicals R3 to R5 may be for example a methylsulphonyl, ethylsulphonyl, propylsulphonyl, isopropylsulphonyl, butylsulphonyl, isobutylsulphonyl, sec-butylsulphonyl, tert-butylsulphonyl, pentylsulphonyl, isopentylsulphonyl, (2-methylbutyl)sulphonyl, (1-methylbutyl)sulphonyl, (1-ethylpropyl)sulphonyl, neopentylsulphonyl, (1,1-dimethylpropyl)sulphonyl, hexylsulphonyl, (4-methylpentyl)-sulphonyl, (3-methylpentyl)sulphonyl, (2-methylpentyl)sulphonyl, (1-methylpentyl)-sulphonyl, (1-ethylbutyl)sulphonyl, (2-ethylbutyl)sulphonyl, (3,3-dimethylbutyl)sulphonyl, (2,2-dimethylbutyl)sulphonyl, (1,1-dimethylbutyl)sulphonyl, (2,3-dimethylbutyl)sulphonyl, (1,3-dimethylbutyl)sulphonyl or a (1,2-dimethylbutyl)sulphonyl group.

The C₃-C₇-cycloalkylsulphonyl groups for the radicals R3 to R5 may be for example a cyclopropylsulphonyl, cyclobutylsulphonyl, cyclopentylsulphonyl, cyclohexylsulphonyl or cycloheptylsulphonyl group.

In the C₃-C₇-cycloalkyl-C₁-C₆-alkylenesulphonyl groups of the radicals R3 to R5 it is possible for each of the C₃-C₇-cycloalkyl groups of the C₃-C₇-cycloalkyl-C₁-C₆-alkylene-sulphonyl groups, for example of a cyclopropyl, cyclobutyl, cyclopentyl, cyclohexyl or cycloheptyl group, to be combined independently of one another with each C₁-C₆-alkylenesulphonyl group, for example with a methylenesulphonyl, ethylenesulphonyl, propylenesulphonyl, butylenesulphonyl, pentylenesulphonyl, hexylenesulphonyl group.

The C₁-C₆-alkylaminosulphonyl groups for the radicals R3 to R5 may be for example a methylaminosulphonyl, ethylaminosulphonyl, propylaminosulphonyl, isopropylaminosulphonyl, butylaminosulphonyl, isobutylaminosulphonyl, sec-butylaminosulphonyl, tert-butylaminosulphonyl, pentylaminosulphonyl, isopentylaminosulphonyl, (2-methylbutyl)-aminosulphonyl, (1-methylbutyl)aminosulphonyl, (1-ethylpropyl)aminosulphonyl, neopentylaminosulphonyl, (1,1-dimethylpropyl)aminosulphonyl, hexylaminosulphonyl, (4-methylpentyl)aminosulphonyl, (3-methylpentyl)aminosulphonyl, (2-methylpentyl)-aminosulphonyl, (1-methylpentyl)aminosulphonyl, (1-ethylbutyl)aminosulphonyl, (2-ethylbutyl)aminosulphonyl, (3,3-dimethylbutyl)aminosulphonyl, (2,2-dimethylbutyl)-aminosulphonyl, (1,1-dimethylbutyl)aminosulphonyl, (2,3-dimethylbutyl)aminosulphonyl, (1,3-dimethylbutyl)aminosulphonyl or a (1,2-dimethylbutyl)aminosulphonyl group.

In the di(C₁-C₆-alkyl)aminosulphonyl groups for the radicals R4 to R6, each of the two C₁-C₆-alkyl radicals on the nitrogen atom of the di(C₁-C₆-alkyl)aminosulphonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

The (C₃-C₇-cycloalkyl)aminosulphonyl groups for the radicals R3 to R5 may be for example a cyclopropylaminosulphonyl, cyclobutylaminosulphonyl, cyclopentylaminosulphonyl, cyclohexylaminosulphonyl or cycloheptylaminosulphonyl group.

In the di(C₃-C₇-cycloalkyl)aminosulphonyl groups for the radicals R3 to R5, each of the two C₃-C₇-cycloalkyl radicals on the nitrogen atom of the di(C₃-C₇-cycloalkyl)amino-sulphonyl group may be independently of one another for example a cyclopropyl, cyclobutyl, cyclopentyl, cyclohexyl or cycloheptyl group.

In the C₃-C₇-cycloalkyl-C₁-C₆-alkyleneaminosulphonyl groups of the radicals R3 to R5, each of the C₃-C₇-cycloalkyl groups of the C₃-C₇-cycloalkyl-C₁-C₆-alkyleneamino-sulphonyl groups, for example of a cyclopropyl, cyclobutyl, cyclopentyl, cyclohexyl or cycloheptyl group, can be combined independently of one another with each C₁-C₆-alkyleneaminosulphonyl group, for example with a methyleneaminosulphonyl, ethyleneaminosulphonyl, propyleneaminosulphonyl, butyleneaminosulphonyl, pentyleneaminosulphonyl, hexyleneaminosulphonyl group.

The C₁-C₆-alkylsulphonylamido groups for the radicals R3 to R5 may be for example a methylsulphonylamido, ethylsulphonylamido, propylsulphonylamido, isopropylsulphonylamido, butylsulphonylamido, isobutylsulphonylamido, sec-butylsulphonylamido, tert-butylsulphonylamido, pentylsulphonylamido, isopentylsulphonylamido, (2-methylbutyl)-sulphonylamido, (1-methylbutyl)sulphonylamido, (1-ethylpropyl)sulphonylamido, neo-pentylsulphonylamido, (1,1-dimethylpropyl)sulphonylamido, hexylsulphonylamido, (4-methylpentyl)sulphonylamido, (3-methylpentyl)sulphonylamido, (2-methylpentyl)-sulphonylamido, (1-methylpentyl)sulphonylamido, (1-ethylbutyl)sulphonylamido, (2-ethylbutyl)sulphonylamido, (3,3-dimethylbutyl)sulphonylamido, (2,2-dimethylbutyl)-sulphonylamido, (1,1-dimethylbutyl)sulphonylamido, (2,3-dimethylbutyl)sulphonylamido, (1,3-dimethylbutyl)sulphonylamido or a (1,2-dimethylbutyl)sulphonylamido group.

In the -N(C₀-C₆-alkyl)-C(O)-C₁-C₆-alkyl groups of the radicals R3 to R5, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₁-C₆-alkyl group on the carbonyl group of the amide, for example with a methyl, ethyl, propyl, iso-propyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N-(C₀-C₆-alkyl)-C(O)-C₃-C₇-cycloalkyl groups of the radicals R3 to R5, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the carbonyl group of the amide, for example with a cyclopropyl, cyclobutyl, cyclopentyl, cyclohexyl or cycloheptyl group.

In the -N(C₀-C₆-alkyl)-C(O)-N-di(C₀-C₆-alkyl) groups of the radicals R3 to R5, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-O-(C₀-C₆-alkyl) groups of the radicals R3 to R5, both (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-NH-(C₃-C₇-cycloalkyl) groups of the radicals R3 to R5, each of the (C₀-C₆-alkyl) groups on the nitrogen atom of the -N(C₀-C₆-alkyl)-C(O)-NH-(C₃-C₇-cycloalkyl) groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₃-C₇-cycloalkyl group on the terminal nitrogen atom of the urea, for example with a cyclopropyl, cyclobutyl, cyclopentyl, cyclohexyl or cycloheptyl group.

In the -N(C₀-C₆-alkyl)-SO₂-(C₁-C₆-alkyl) groups of the radicals R3 to R5, each of the (C₀-C₆-alkyl) groups on the nitrogen atom of the -N(C₀-C₆-alkyl)-SO₂-(C₁-C₆-alkyl) group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₁-C₆-alkyl group on the sulphonyl group of the sulphonamide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-SO₂-C₃-C₇-cycloalkyl groups of the radicals R3 to R5, each of the (C₀-C₆-alkyl) groups on the nitrogen atom of the -N(C₀-C₆-alkyl)-SO₂-C₃-C₇-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the sulphonyl group, for example with a cyclopropyl, cyclobutyl, cyclopentyl, cyclohexyl or cycloheptyl group.

In the -N(C₀-C₆-alkyl)-SO₂-N-di(C₀-C₆-alkyl) groups of the radicals R3 to R5, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-SO₂-NH-(C₃-C₇)-cycloalkyl groups of the radicals R3 to R5, the C₀-C₆-alkyl group of the -N(C₀-C₆-alkyl)-SO₂-NH-(C₃-C₇)-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group, for example with a cyclopropyl, cyclobutyl, cyclopentyl, cyclohexyl or cycloheptyl group.

In the -C(O)-N(H)-C₂-C₆-alkylene-(C₁-C₆-alkyl)amine groups of the radicals R3 to R5, each of the C₂-C₆-alkylene groups on the nitrogen atom of the -C(O)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -C(O)-N(H)-C₂-C₆-alkylene-[di(C₁-C₆-alkyl)]amine groups of the radicals R3 to R5, each of the C₂-C₆-alkylene groups on the nitrogen atom of the -C(O)-N(H)-C₂-C₆-alkylene-[di(C₁-C₆-alkyl)]amine group, for example an ethylene, propylene, butylene, pentylene or hexylene group, may be combined independently of one another with each of the two identically or different C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -C(O)-N(H)-C₂-C₆-alkylene-(C₃-C₇-cydoalkyl)amine groups of the radicals R3 to R5, each of the (C₂-C₆-alkylene) groups of the -C(O)-N(H)-C₂-C₆-alkylene-(C₃-C₇-cycloalkyl)amine group, for example an ethylene, propylene, butylene, pentylene or hexylene group, may be combined independently of one another with each C₃-C₇-cycloalkyl group on the amine, for example with a cyclopropyl, cyclobutyl, cyclopentyl, cyclohexyl or cycloheptyl group.

In the -C(O)-N(H)-C₂-C₆-alkylene-(C₃-C₇-cycloalkyl-C₁-C₆-alkylene)amine groups of the radicals R3 to R5, each of the (C₂-C₆-alkylene) groups of the -C(O)-N(H)-C2-C6-alkylene-(C3-C6-cycloalkyl-C1-C6-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylene or cycloheptylhexylene group.

In the -S(O₂)-N(H)-C₂-C₆-alkylene-(C₁-C₆-alkyl)amine groups of the radicals R3 to R5, the (C₂-C₆-alkylene) groups of the -S(O₂)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-[di(C₁-C₆-alkyl)]amine groups of the radicals R3 to R5, the C₂-C₆-alkylene group of the -S(O₂)-N(H)-C₂-C₆-alkylene-[di(C₁-C₆-alkyl)]amine group, for example an ethylene, propylene, butylene, pentylene or hexylene group, may be combined independently of one another with each of the two C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-(C₃-C₇-cycloalkyl)amine groups of the radicals R3 to R5, the C₂-C₆-alkylene group of the -S(O₂)-N(H)-C₂-C₆-alkylene-(C₃-C₇-cycloalkyl)-amine group, for example an ethylene, propylene, butylene, pentylene or hexylene group, may be combined independently of one another with each C₃-C₇-cycloalkyl group on the amino group, for example with a cyclopropyl, cyclobutyl, cyclopentyl, cyclohexyl or cycloheptyl group.

In the -S(O₂)-N(H)-C₂-C₆-alkylene-(C₃-C₇-cycloalkyl-C₁-C₆-alkylene)amine groups of the radicals R3 to R5, each C₂-C₆-alkylene group of the -S(O₂)-N(H)-C₂-C₆-alkylene-(C₃-C₇-cycloalkyl-C₁-C₆-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylen or cycloheptylhexylene group.

In the -O-C₂-C₆-alkylene-(C₁-C₆-alkyl)amine groups of the radicals R3 to R5, the C₂-C₆-alkylene group of the -O-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -O-C₂-C₆-alkylene-[di(C₁-C₆-alkyl)]amine groups of the radicals R3 to R5, the C₂-C₆-alkylene group of the -O-C₂-C₆-alkylene-[di(C₁-C₆-alkyl)]amine group, for example an ethylene, propylene, butylene, pentylene or hexylene group, may be combined independently of one another with two freely selectable C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

Compounds preferred according to the present invention are N-acyl homotryptophanols of the formula II 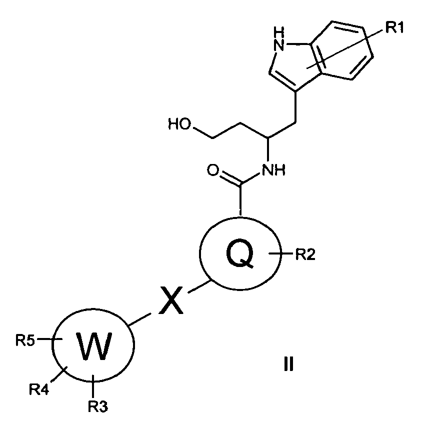 in which R1, R2, R3, R4, R5, X, Q and W have the same meaning as defined in formula I.

Compounds particularly preferred according to the present invention are N-acyl homotryptophanols of the formula III 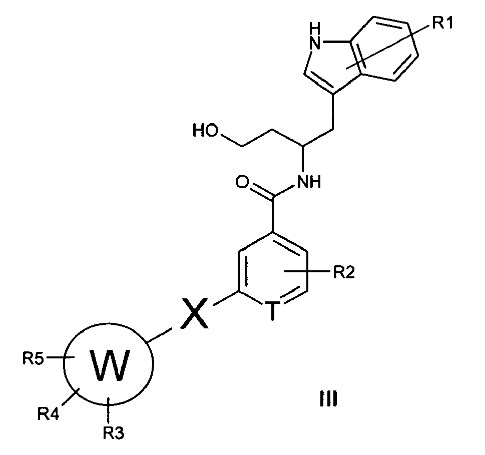 in which
- T: may be a nitrogen atom or a CH group;
and R1, R2, R3, R4, R5, X, and W have the same meaning as defined in formula I.

Compounds likewise particularly preferred according to the present invention are N-acyl homotryptophanols of the formula IV 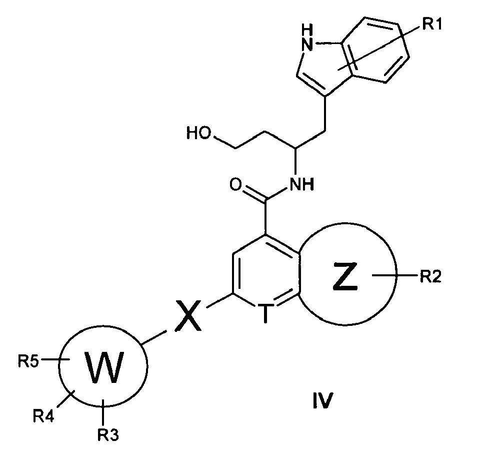 in which
- T: may be a nitrogen atom or a CH group;
- Z: is a cycloalkylen, cycloalkenylen, heterocycloalkylen or heterocycloalkenylen group or a a monocyclic aryl or a monocyclic heteroaryl group;
- R1, R2, R3, R4, R5, X and W: have the same meaning as defined in formula I,
where
- R2: may substitute one or more positions of the radical Z.

The following compounds are very particularly preferred:

| | |
|---|---|
| **1** | 6-Methoxy-2-(3,4,5-trimethoxy-phenyl)-quinoline-4-carboxylic acid [(R)-3-hydroxy-1-(1 H-indol-3-ylmethyl)-propyl]-amide; |
| **2** | 4-Ethoxy-3'-methoxy-biphenyl-3-carboxylic acid [(R)-3-hydroxy-1-(1 H-indol-3-ylmethyl)-propyl]-amide; |
| **3** | 3'-Chloro-4-isopropoxy-biphenyl-3,4'-dicarboxylic acid 3-{[(R)-3-hydroxy-1-(1 H-indol-3-ylmethyl)-propyl]-amide} 4'-methylamide; |
| **4** | 4-Isopropoxy-3',4'-dimethoxy-biphenyl-3-carboxylic acid [(R)-3-hydroxy-1-(1 H-indol-3-ylmethyl)-propyl]-amide; |
| **5** | N-[(R)-3-Hydroxy-1-(1H-indol-3-ylmethyl)-propyl]-2-isopropoxy-5-(2-methoxy-phenylethynyl)-benzamide; |
| **6** | 4-Ethoxy-3'-methoxy-biphenyl-3-carboxylic acid ((R)-3-hydroxy-1-naphtha)en-1-ylmethyl-propyl)-amide; |
| **7** | 6-Methoxy-2-(3,4,5-trimethoxy-phenyl)-quinoline-4-carboxylic acid ((R)-3-hydroxy-1-naphthalen-1-ylmethyl-propyl)-amide; |
| **8** | 6-Methoxy-2-(3,4,5-trimethoxy-phenyl)-quinoline-4-carboxylic acid ((R)-3-hydroxy-1-naphthalen-2-ylmethyl-propyl)-amide; |
| **9** | 6-Methoxy-2-(3,4,5-trimethoxy-phenyl)-quinoline-4-carboxylic acid ((R)-1-benzo[b]thiophen-3-ylmethyl-3-hydroxy-propyl)-amide; |
| **10** | 6-Methoxy-2-(3,4,5-trimethoxy-phenyl)-quinoline-4-carboxylic acid ((R)-3-hydroxy-1-pyridin-3-ylmethyl-propyl)-amide; |
| **11** | 6-Methoxy-2-(3,4,5-trimethoxy-phenyl)-quinoline-4-carboxylic acid ((R)-3-hydroxy-1-thiophen-2-ylmethyl-propyl)-amide; |
| **12** | 6-Methoxy-2-(3,4,5-trimethoxy-phenyl)-quinoline-4-carboxylic acid ((R)-3-hydroxy-1-thiophen-3-ylmethyl-propyl)-amide; |
| **13** | 6-Methoxy-2-(3,4,5-trimethoxy-phenyl)-quinoline-4-carboxylic acid ((S)-1-furan-2-ylmethyl-3-hydroxy-propyl)-amide; |
| **14** | 6-Methoxy-2-(3,4,5-trimethoxy-phenyl)-quinoline-4-carboxylic acid [(S)-3-hydroxy-1-(1H-indol-3-ylmethyl)-propyl]-amide; |
| **15** | 6-Methoxy-2-(3,4,5-trimethoxy-phenyl)-quinoline-4-carboxylic acid ((R)-1-benzyl-3-hydroxy-propyl)-amide; |
| **16** | 4-Ethoxy-3'-methoxy-biphenyl-3-carboxylic acid ((R)-1-benzyl-3-hydroxypropyl)-amide; |
| **17** | 6-Methoxy-2-(3,4,5-trimethoxy-phenyl)-quinoline-4-carboxylic acid ((S)-1-benzyl-3-hydroxy-propyl)-amide. |

### Pharmacological investigations

### HTRF assay for measuring cAMP in cells

The method is based on a competitive immunoassay between native cAMP, which has been produced by the cells, and cAMP which is labelled with XL665. The tracer binding was visualized by a monoclonal antibody, anti-cAMP labelled with cryptate [HTRF = homogeneous time-resolved fluorescence].

The specific signal is inversely proportional to the cAMP concentration of the samples employed.

The 665nm/ 620nm fluorescence ratio was evaluated.

*The following material was used: 96-well plates for the tissue culture, 96-well plates with black edge and black base (e.g. Fluotrac 600 from Greiner), 96-well plates for the substance dilutions of polypropylene and cAMP Femtomolar (4000wells Kit, CIS Bio International # 62AM1PEC).*

The following reagents were used: BSA (bovine serum albumin) Fraction V protease-free, IBMX (3-isobutyl-1-methylxanthine), hFSH (human follicle stimulating hormone), Triton X-100 analytical grade, potassium fluoride analytical grade, G 418 (Geneticin) and Accutase.
Buffer 1 (washing and testing buffer) contained PBS, 1 mM CaCl2, 1 mM MgCl₂, 0.2% glucose; 0.1% BSA, 1 mM IBMX.
Buffer 2 (2x lysis buffer) contained 1% Triton X-100 in PBS (without CaCl₂ and MgCl₂).
Buffer 3 (assay buffer) contained 50 mM potassium phosphate buffer (pH 7.0); 800 mM potassium fluoride; 0.2% BSA (always added fresh).

### Procedure:

On day 1, the cells were seeded in 96-well plates (3x10⁴ cells per well hFSHR clone 16 cells (CHO cells stably transfected with the human FSH receptor in 150 µl of medium). The next day, test substance dilutions were made up. For this purpose, all the substances were diluted in ice-cold buffer 1 (with or without hFSH), and the substance dilutions were placed on ice until applied to the cells.

The cell supernatant was then aspirated off, and the cells were washed 2x with 200 µl of buffer 1. The cells were treated with 60 µl of the appropriate substance concentrations at 37°C for 2h. The cells were then lysed with 60 µl of buffer 2 (put onto the supernatant) (on a plate shaker at RT for 30 min).

The test conjugates (XL-665 and anti-cAMP cryptate) were diluted in buffer 3 in accordance with the manufacturers' information. The actual mixture for measurement was pipetted into a black 96-well plate (in each case 15 µl of the cell lysate diluted with 35 µl of buffer 1; firstly 25 µl of XL-665 conjugate were pipetted and, after 10 min, 25 µl of the anti-cAMP cryptate were added). This is followed by incubation at RT for 90 minutes. The measurement was carried out in a PheraStar (BMG).

### Tissue culture conditions

| | |
|---|---|
| 1) hFSHr clone 16 | Ham's F12 |
| | PSG |
| | 10% FCS |
| | 700 µg/ml G 418 (Geneticin) from PAA. |

Dose-effect curve (hFSH) for the human receptor: 1 e-8, 3e-9, 1 e-9, 3e-10, 1 e-1 0, 3e-11, 1 e-11, 3e-12 mol/l.

The test substances were employed in suitable dilutions in the absence (test for agonism) and in the presence of 1e-9 mol/l hFSH.

### Evaluation

The values of the well ratio were averaged and then entered directly in SigmaPlot versus the concentrations. The maximum and minimum values were determined for each plate, and half the difference is to be regarded as IC₅₀.

The test results (Table 1) show that the compounds according to the invention have an FSH-modulatory effect.

**Table 1. FSH antagonistic and / or agonistic effect of selected compounds in the HTRF assay**

| **Compound [Ex. #]** | **IC₅₀** | **EC₅₀** |
|---|---|---|
| **2** | **300 nM** | **>10µM** |
| **3** | **25 nM** | **> 10 µM** |
| **5** | **150 nM** | **>10 µM** |
| **8** | **>10 µM** | **7 µM** |
| **9** | **> 10 µM** | **10 µM** |
| **12** | **> 10 µM** | **5 µM** |
| **14** | **> 10 µM** | **6 µM** |
| **17** | **> 10 µM** | **8 µM** |

Being modulators i.e. antagonists and / or agonists of the FSH receptor, compounds of the general formula I or pharmaceutically acceptable salts thereof can thus be used for the manufacture of medicaments to be used for fertility regulation in male and / or in a female animals, in particular in men and / or women; as well as for the treatment and / or prevention of osteoporosis.

Fertility regulation in men or women include fertility control (i.e. male fertility control, female contraception) as well as pro-fertility measures (increasing fertility) in men or women.

### Dosage

Satisfactory results are generally to be expected if the daily doses comprise a range from 5 µg to 50 mg of the compound according to the invention per kg of body weight. A recommended daily dose for larger mammals, for example humans, is in the range from 10 µg to 30 mg per kg of body weight. Suitable dosages for the compounds according to the invention are from 0.005 to 50 mg per day per kg of body weight, depending on the age and constitution of the patient, it being possible to administer the necessary daily dose by single or multiple delivery.

Pharmaceutical products based on the novel compounds are formulated in a manner known per se by processing the active ingredient with the carrier substances, fillers, substances which influence disintegration, binders, humectants, lubricants, absorbents, diluents, test modifiers, colorants etc. which are used in pharmaceutical technology, and converting into the desired administration form. Reference should be made in this connection to Remington's Pharmaceutical Science, 15th ed. Mack Publishing Company, East Pennsylvania (1980).

Suitable for oral administration are in particular tablets, coated tablets, capsules, pills, powders, granules, pastilles, suspensions, emulsions or solutions. Preparations for injection and infusion are possible for parenteral administration. Appropriately prepared crystal suspensions can be used for intraarticular injection. Aqueous and oily solutions for injection or suspensions and corresponding depot preparations can be used for intramuscular injection. The novel compounds can be used for rectal administration in the form of suppositories, capsules, solutions (e.g. in the form of enemas) and ointments both for systemic and for local therapy. Formulations possible for topical application are gels, ointments, greasy ointments, creams, pastes, dusting powders, milk and tinctures. The dosage of the compounds of the general formula I in these preparations should be 0.01% - 20% in order to achieve an adequate pharmacological effect. Topical use can also take place by means of a transdermal system, for example a patch.

The invention likewise encompasses the compounds according to the invention of the general formula I as therapeutic active ingredient. The invention further includes the compounds according to the invention of the general formula I as therapeutic active ingredients together with pharmaceutically suitable and acceptable excipients and carriers. The invention likewise encompasses a pharmaceutical composition which comprises one of the pharmaceutically active compounds according to the invention or mixture thereof and a pharmaceutically suitable salt or pharmaceutically suitable excipients and carriers.

The present invention therefore also relates to pharmaceutical compositions which comprise at least one compound of the general formula I, where appropriate together with pharmaceutically suitable excipients and/or carriers.

Suitable for forming pharmaceutically suitable salts of the compounds according to the invention of the general formula I are, by methods known to the skilled person, as inorganic acids inter alia hydrochloric acid, hydrobromic acid, sulphuric acid and phosphoric acid, nitric acid, as carboxylic acids inter alia acetic acid, propionic acid, hexanoic acid, octanoic acid, decanoic acid, oleic acid, stearic acid, maleic acid, fumaric acid, succinic acid, benzoic acid, ascorbic acid, oxalic acid, salicylic acid, tartaric acid, citric acid, lactic acid, glycolic acid, malic acid, mandelic acid, cinnamic acid, glutamic acid, aspartic acid, and as sulphonic acids inter alia methanesulphonic acid, ethanesulphonic acid, toluenesulphonic acid, benzenesulphonic acid and naphthalenesulphonic acid.

These pharmaceutical compositions and medicaments may be intended for oral, rectal, subcutaneous, transdermal, percutaneous, intravenous or intramuscular administration. They comprise besides conventional carriers and/or diluents at least one compound of the general formula I.

The medicaments of the invention are produced using the customary solid or liquid carriers or diluents and the excipients customarily used in pharmaceutical technology, in accordance with the desired mode of administration with a suitable dosage in a known manner. The preferred preparations consist of a dosage form which is suitable for oral administration. Examples of such dosage forms are tablets, film-coated tablets, sugar-coated tablets, capsules, pills, powders, solutions or suspensions or else depot forms.

The pharmaceutical compositions which comprise at least one of the compounds according to the invention are preferably administered orally.

Parenteral preparations such as solutions for injection are also suitable. Preparations which may also be mentioned for example are suppositories.

Appropriate tablets can be obtained for example by mixing the active ingredient with known excipients, for example inert diluents such as dextrose, sugar, sorbitol, mannitol, polyvinylpyrrolidone, disintegrants such as maize starch or alginic acid, binders such as starch or gelatine, lubricants such as magnesium stearate or talc and/ or agents to achieve a depot effect such as carboxylpolymethylene, carboxylmethylcellulose, cellulose acetate phthalate or polyvinyl acetate. The tablets may also consist of a plurality of layers.

Correspondingly, coated tablets can be produced by coating cores which have been produced in analogy to the tablets with agents normally used in tablet coatings, for example polyvinylpyrrolidone or shellac, gum Arabic, talc, titanium oxide or sugar. The tablet coating may also consist of a plurality of layers, it being possible to use the excipients mentioned above for tablets.

Solutions or suspensions with the compounds according to the invention of the general formula I may additionally comprise taste-improving agents such as saccharin, cyclamate or sugar and, for example, flavourings such as vanillin or orange extract. They may additionally comprise suspending aids such as sodium carboxymethylcellulose or preservatives such as p-hydroxybenzoates.

Capsules comprising the compounds of the general formula I can be produced for example by the compound(s) of the general formula I being mixed with an inert carrier such as lactose or sorbitol and encapsulated in gelatine capsules.

Suitable suppositories can be produced for example by mixing with carriers intended for this purpose, such as neutral fats or polyethylene glycol or derivatives thereof.

The present invention also relates to processes for preparing the compounds according to the invention.

Compounds of the general formula I can be prepared as shown in Scheme 1 by an amide-formation reaction between the amino alcohol derivative V and the carboxylic acid VI. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (*N*-[3-(dimethylamino)propyl]-*N'*-ethylcarbodiimide hydrochloride) / HOBt (1-hydroxy-1*H*-benzotriazole). It is possible as alternative for the carboxylic acid VI to be converted, where appropriate in the presence of a base, into the carbonyl chloride and reacted with the aminoalcohol V to give the product of the general formula I.

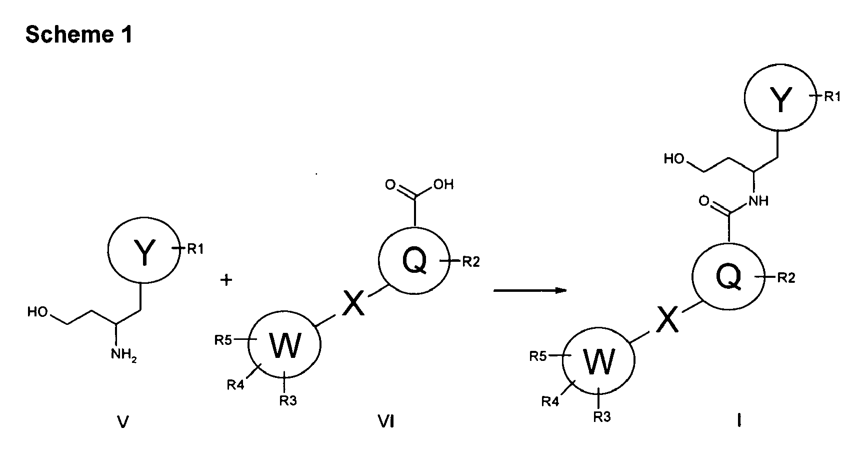

Compounds of general formula II can be prepared as shown in Scheme 2 by an amide-formation reaction between the aminoalcohol derivative VII and carboxylic acid VI. Reagents suitable for this purpose are all known peptide-coupling reagents which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (N-[3-(dimethylamino)propyl]-N'-ethylcarbodiimide hydrochloride) / HOBt (1-hydroxy-1*H*-benzotriazole). It is possible as alternative for the carboxylic acid VI to be converted, where appropriate in the presence of a base, into the carbonyl chloride and reacted with the aminoalcohol VII to give the product of the general formula II.

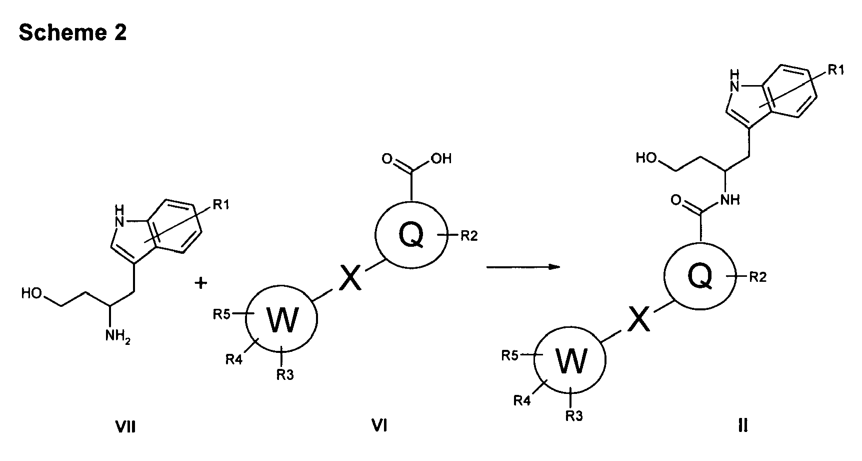

Compounds of general formulae III and IV can be prepared as shown in Scheme 3 by an amide-formation reaction between the aminoalcohol derivative VII and the appropriate carboxylic acid VIII or IX. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (N-[3-(dimethylamino)propyl]-N'-ethylcarbodiimide hydrochloride) / HOBt (1-hydroxy-1*H*-benzotriazole). It is possible as alternative for the carboxylic acids VIII or IX to be converted, where appropriate in the presence of a base, into the carbonyl chloride and reacted with the aminoalcohol VII to give the product of the general formulae III or IV.

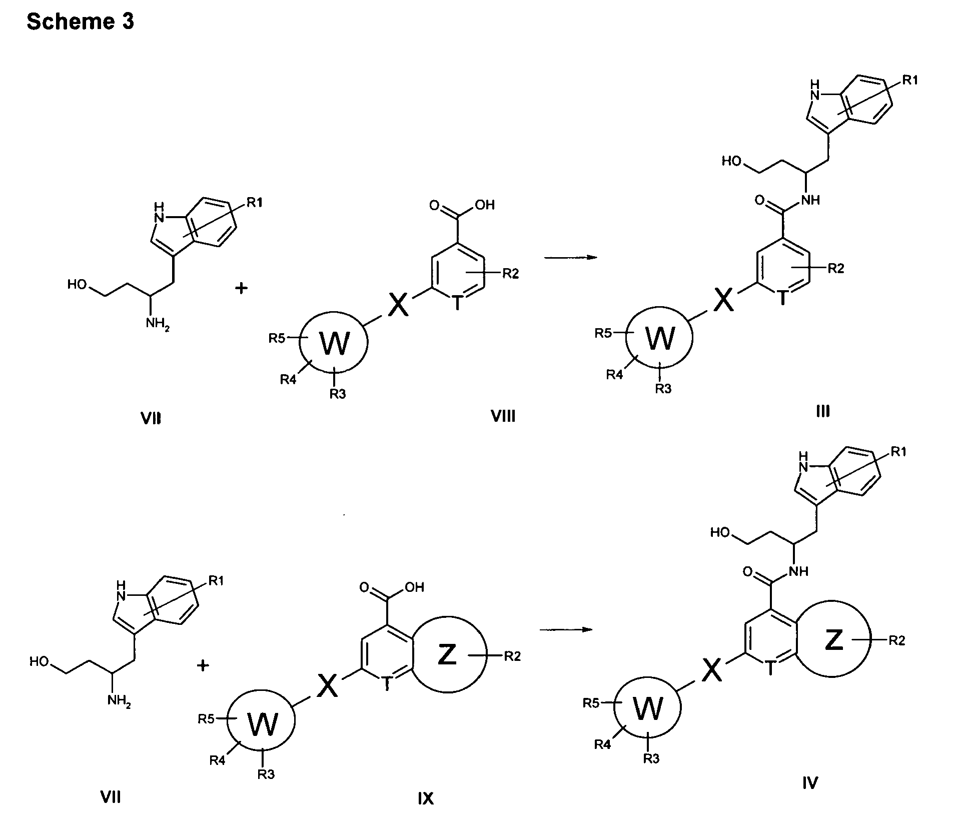

The compounds according to the invention of the general formula I can be prepared as described below.

### Abbreviations used:

| | |
|---|---|
| ACN | Acetonitrile |
| DIBAC | Diisobutylaluminium hydride |
| DMF | N,N-Dimethylformamide |
| EDC | *N*-Ethyl-*N'*-(3-dimethylaminopropyl)carbodiimide |
| EtOH | Ethanol |
| HATU | O-(7-Azabenzotriazol-1-yl)-N, N, N',N'-tetramethyluronium hexafluorophosphate |
| FMOC | (9H-Fluoren-9-ylmethoxy)carbonyl |
| HOBt | 1-Hydroxy-1*H-*benzotriazole |
| MeCN | Acetonitrile |
| MeOH | Methanol |
| MTBE | Methyl tert-butyl ether |
| NMM | 4-methylmorpholine |
| NMP | N-Methylpyrrolidinone |
| Rf | Reflux |
| RT | Room temperature |
| TBAF | Tetrabutylammonium fluoride |
| TFA | Trifluoroacetic acid |
| THF | Tetrahydrofuran |

Compounds of the general formula I can in principle be prepared as shown in Scheme 4 by an amide-formation reaction between a aminoalcohol derivative V and a carboxylic acid VI. The reagents typically used for the coupling are EDC and HOBt. The aminoalcohol derivatives of the formula V can be purchased or are known from the literature. 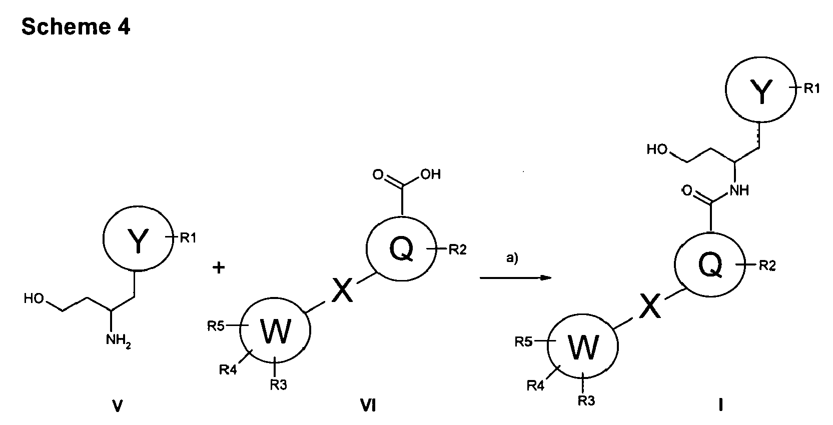 **Reagents**: a) HOBt, EDC, DMF, base, RT.

The amino alcohols V can be prepared according to scheme 5 from the corresponding β-amino acids which are known from the literature or commercially available. For a review see Seebach et al. in Biopolymers 2004, 76 (3), pages 206-243. The amino group of the amino acids is protected with a Boc group and the carboxylic acid XI is then reduced to the corresponding alcohol XII. Subsequent cleavage of the Boc group with trifluoroacetic acid yields the amino alcohols V. 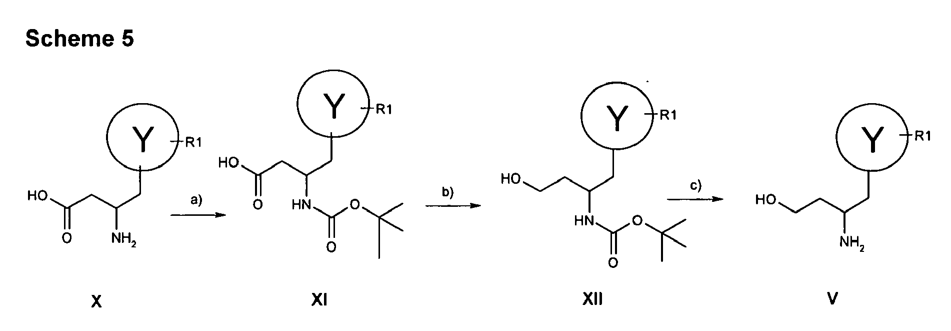 **Reagents:** a) (Boc)₂O; b) ClC(O)OCH₃, N-methyl morpholine THF; lithium borohydride, THF; c) TFA, CH₂Cl₂.

The carboxylic acids of the general formula VI can be prepared as shown in Scheme 6 by a Suzuki reaction between a boronic acid XIII or XVII and a halogen compound XIV or XVI (Hal = I, Br, Cl) with subsequent hydrolysis of ester XV. 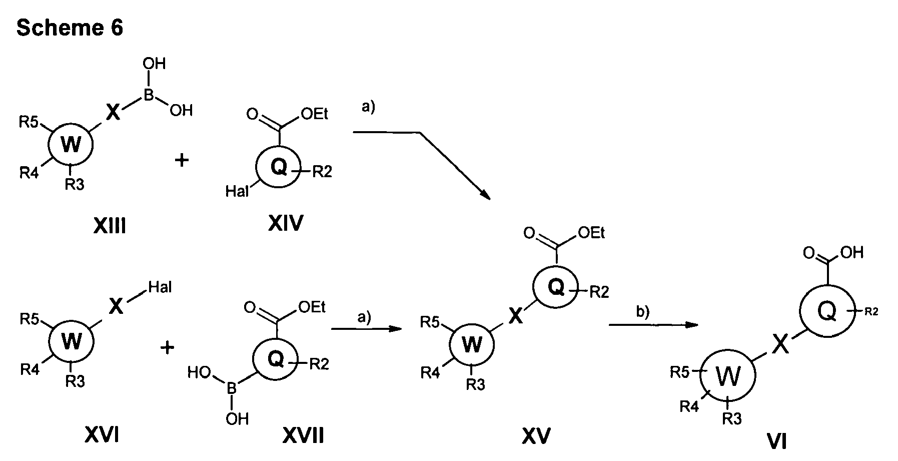 **Reagents:** a) TBAF, Pd(PPh₃)₄, THF, Rf; b) KOH, MeOH;.

The carboxylic acid derivatives of formula XX can in principle be prepared according to Scheme 7 via a Sonogashira type coupling of acetylenes XVI II or XXII with their corresponding aryl halides XIV or XXI with subsequent hydrolysis of the resulting carboxylic ester XIX. 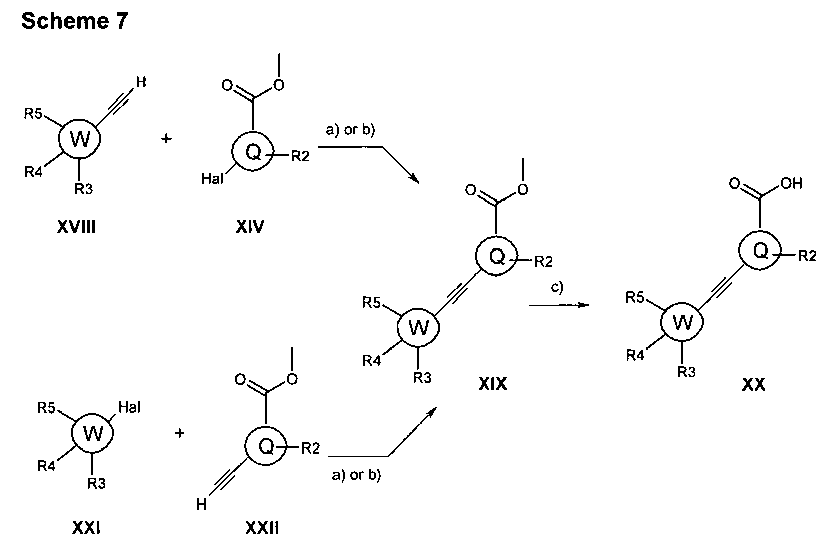 **Reagents:** a) Pd(PPh₃)₂Cl₂, TBAF, neat or THF; b) Pd(PPh₃)₂Cl₂, Cul, Et₂NH; c) KOH, MeOH.

Carboxylic acids of the formula XXV can be prepared as shown in Scheme 8 in a socalled Pfitzinger reaction from a methyl ketone XXlll and an isatin derivative XXIV. 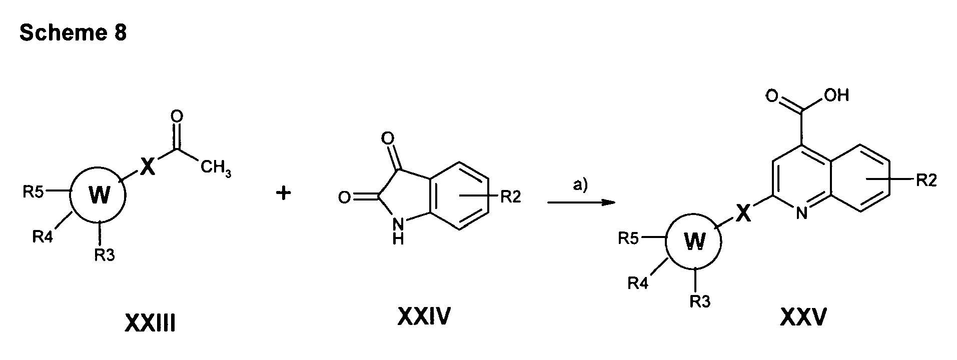 **Reagents:** a) KOH, EtOH.

### Synthesis of the compounds according to the invention

### Example 1

### 6-Methoxy-2-(3,4,5-trimethoxy-phenyl)-quinoline-4-carboxylic acid [(R)-3-hydroxy-1-(1 H-indol-3-ylmethyl)-propyl]-amide

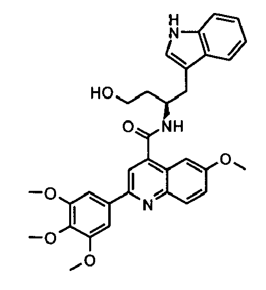

1 a) 6-Methoxy-2-(3,4,5-trimethoxy-phenyl)-quinoline-4-carboxylic acid
   2.05 mmol of potassium hydroxide were slowly added to a stirred solution of 0.68 mmol of 5-Methoxyisatin in 7 ml of ethanol and 0.82 mmol of 3,4,5-Trimethoxyacetophenone. After the addition was complete, the mixture was stirred at 80°C for six hours. The solution was cooled and then the ethanol was removed in vacuo. The residue was taken up in water and acidified with 2 ml of 1 M aqueous hydrochloric acid. The aqueous phase was extracted with ethyl acetate. The resulting organic phase was dried over magnesium sulphate, filtered and concentrated in vacuo. Flash chromatography resulted in 145 mg of the target compound. **ESI-MS [M+1]** = 370.
1b) (R)-3-Amino-4-(1H-indol-3-yl)-butan-1-ol
   Commercially available (R)-3-tert-Butoxycarbonylamino-4-(1H-indol-3-yl)-butyric acid (400 mg), N-Methylmorpholine (0.21 ml), Methylchloroformiat (0.15 ml) were dissolved in THF (16 ml) and stirred at -10°C for one hour. A solution of lithium borohydride (2M in THF, 1.26 ml) was added slowly at -10°C, the reaction was allowed to warm to ambient temperature, water was added (4 ml) and the reaction mixture was neutralized with aqueous HCl (1N). THF was distilled off under reduced pressure. The reaction was extracted with ethyl acetate (3 x 50 ml) and water (50 ml) and the combined organic layers were dried over magnesium sulphate. After flash chromatography 278 mg of [(R)-3-Hydroxy-1-(1H-indol-3-ylmethyl)-propyl]-carbamic acid tert-butyl ester was obtained. 3-Hydroxy-1-(1H-indol-3-ylmethyl)-propyl]-carbamic acid tert-butyl ester (278 mg) was dissolved in dichloromethane (6 ml) and trifluoroacetic acid (0.35 ml) and the reaction mixture was stirred at ambient temperature overnight. The pH value was adjusted by the addition of aqueous sodium bicarbonate and NaOH to pH =12 and the reaction was stirred at 60°C overnight, then cooled to ambient temperature and extracted with ethyl acetate (3 x 50 ml) and water (50 ml). The combined organic layers were washed several times with water until pH = 9 and then dried over magnesium sulphate. The solvent was removed under vacuum to yield 174 mg of the title compound which was used without further purification. **ESI-MS [M+1]** = 205.
1c) 6-Methoxy-2-(3,4,5-trimethoxy-phenyl)-quinoline-4-carboxylic acid [(R)-3-hydroxy-1-(1 H-indol-3-ylmethyl)-propyl]-amide
   A solution of 6-Methoxy-2-(3,4,5-trimethoxy-phenyl)-quinoline-4-carboxylic acid (73 mg), (R)-3-Amino-4-(1H-indol-3-yl)-butan-1-ol (40 mg), HOBt monohydrate (30 mg) and EDC (38 mg) in DMF (3 ml) was stirred overnight at ambient temperature. The reaction mixture was poured into water and the resulting precipitate was filtered off. After drying the 55 mg of the title compound was obtained. **¹H-NMR (DMSO-*d₆*):** 10.84 s (1 H); 8.67 d (J = 8.2 Hz, 1 H); 8.01 d (J = 9.0 Hz, 1 H); 7.93 s (1 H); 7.66 d (J = 7.8 Hz, 1 H); 7.48 s (2H); 7.43 dd (J = 9.0 Hz / 2.7 Hz, 1 H); 7.38 d (J = 2.7 Hz, 1 H); 7.32 d (J = 8.2 Hz, 1 H); 7.22 d (J = 1.6 Hz, 1 H); 7.04 m (1 H); 6.94 m (1 H); 4.48 m (2H); 3.92 s (6H); 3.76 s (3H); 3.75 s (3H); 3.59 m (2H); 3.07 m (1 H); 3.00 m (1 H); 1.82 m (1 H); 1.77 m (1H).

### Example 2

### 4-Ethoxy-3'-methoxy-biphenyl-3-carboxylic acid [(R)-3-hydroxy-1-(1H-indol-3-ylmethyl)-propyl]-amide

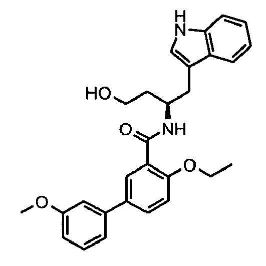

2a) 4-Ethoxy-3'-methoxy-biphenyl-3-carboxylic acid was prepared in analogy to WO 2007/017289, example 39a and 39b.
2b) 4-Ethoxy-3'-methoxy-biphenyl-3-carboxylic acid [(R)-3-hydroxy-1-(1H-indol-3-ylmethyl)-propyl]-amide
A solution of 4-Ethoxy-3'-methoxy-biphenyl-3-carboxylic acid (52 mg), (R)-3-Amino-4-(1H-indol-3-yl)-butan-1-ol (32 mg), HOBt monohydrate (29 mg) and EDC (36 mg) in DMF (2 ml) was stirred overnight at ambient temperature. The reaction mixture was poured into water and the resulting precipitate was filtered off. After drying the 20 mg of the title compound was obtained. **(DMSO-*d₆*):** 10.83 s (1 H); 8.17 d (J = 8.2 Hz, 1 H); 7.97 d (J = 2.3 Hz, 1 H); 7.73 dd (J = 2.3 Hz / 8.6 Hz, 1 H); 7.61 d (J = 7.8 Hz, 1 H); 7.35 m (2H); 7.18 m (4H); 7.04 t (J = 7.4 Hz, 1 H); 6.92 m (2H); 4.43 m (1 H); 4.36 m (1 H); 4.10 m (2H); 3.82 s (3H); 3.51 m (2H); 2.99 m (1 H); 2.92 m (1 H); 1.74 m (1 H); 1.59 m (1 H); 1.24 m (3H).
The following compounds were obtained in analogy to the preparation methods described in detail:

| **Ex.** | **Product; reagents** | **Method analogous to** | **¹H-NMR (400 MHz)** δ **[ppm]** | **Structure** |
|---|---|---|---|---|
| **3** | 3'-Chloro-4-isopropoxy-biphenyl-3,4'-dicarboxylic acid 3-{[(R)-3-hydroxy-1-(1H-indol-3-ylmethyl)-propyl]-amide} 4'-methylamide; | **2** | **(DMSO-*d₆*):** 10.80 s (1H); 8.34 d (J = 4.7 Hz, 1H); 8.12 d (J = 8.3 Hz, 1H); 7.99 d (J = 2.5 Hz, 1H); 7.76 dd (*J* = 8.7 Hz / 2.6 Hz, 1H); 7.70 d (*J* = 1.7 Hz, 1H); 7.59 m (2H); 7.46 d (J = 7.9 Hz, 1H); 7.30 d (*J* = 8.1 Hz, 1H); 7.20 d (*J* = 9.0 Hz, 1H); 7.11 d (J = 2.3 Hz, 1H); 7.02 m (1H); 6.91 m (1H); 4.74 m (1H); 4.40 m (1H); 4.33 s (1H); 3.47 m (2H); 2.97 m (1H); 2.89 m (1H); 2.73 d (J = 4.7 Hz, 1H); 1.74 m (1H); 1.56 m (1H); 1.19 m (6H). | 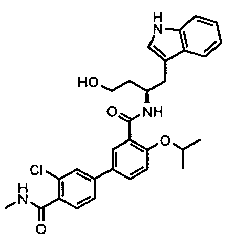 |
| | | | | |
| | *3'-Chloro-4-isopropoxy-4'-methylcarbamoyl-biphenyl-3-carboxylic acid* | | | |
| | | | | |
| | *and* | | | |
| | | | | |
| | *(R)-3-Amino-4-(1H-indol-3-yl)-butan-1-ol* | | | |
| **4** | 4-Isopropoxy-3',4'-dimethoxy-biphenyl-3-carboxylic acid [(R)-3-hydroxy-1-(1 H-indol-3-yl-methyl)-propyl]-amide; | **2** | **(DMSO-*d₆*):** 10.80 s (1H); 8.3 d (*J* = 8.3 Hz, 1H); 7.96 d (*J* = 2.6 Hz, 1H); 7.66 dd (*J* = 8.7 Hz / 2.6 Hz, 1H); 7.59 d (*J* = 7.7 Hz, 1H); 7.30 d (*J* = 8.1 Hz, 1H); 7.14 d (J = 9.0 Hz, 1H); 7.11 m (1H); 7.01 m (1H); 6.91 m (1H); 4.71 m (1H); 4.40 m (1H); 4.34 s (1H); 3.81 s (3H); 3.75 s (3H); 3.47 m (2H); 2.97 m (1H); 2.89 m (1H); 1.74 m (1H); 2.89 m (1H); 1.74 m (1H); 1.56 m (1H); 1.18 m (6H). | 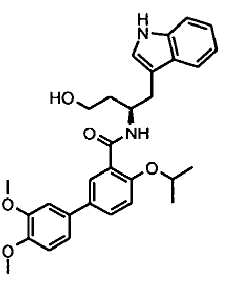 |
| | | | | |
| | *4-Isopropoxy-3',4'-dimethoxy-biphenyl-3-carboxylic acid* | | | |
| | | | | |
| | *and* | | | |
| | | | | |
| | *(R)-3-Amino-4-(1H-indol-3-yl)-butan-1-ol* | | | |

### Example 5

### N-[(R)-3-Hydroxy-1-(1H-indol-3-ylmethyl)-propyl]-2-isopropoxy-5-(2-methoxy-phenylethylnyl)-benzamide

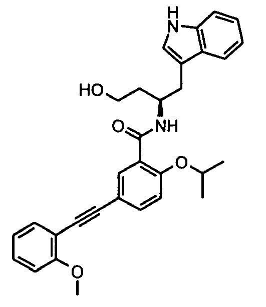

5a) 5-lodo-2-isopropoxy-benzoic acid methyl ester
   A suspension of 5-lodo-2-hydroxy benzoic acid methyl ester (50 g), potassium carbonate (74,6 g) and *iso*-propyl iodide (89,9 ml) in acetone (500 ml) was stirred under reflux overnight. The reaction mixture was allowed to cool down to ambient temperature and the solid was removed by filtration. The filtrate was evaporated and the title compound was obtained in 96 % yield (55,1 g). **¹H-NMR (CDCl₃):** 8.02 d (*J* = 2.3 Hz, 1 H); 7.67 dd (*J* = 2.5 Hz / 8.9 Hz, 1 H); 6.74 d (*J* = 8.9 Hz, 1 H); 4.54 m (1 H); 3.87 s (3H); 1.36 m (6H).
5b) 2-isopropoxy-5-(2-methoxy-phenylethynyl)-benzoic acid methyl ester
   5-lodo-2-isopropoxy-benzoic acid methyl ester (500 mg), 2-methoxy-phenyl acetylene (0.40 ml), palladium dichlorobis(triphenylphosphine) (12 mg) and Cul (6 mg) in diethylamine (10 ml) were stirred at 60°C for 12 hours. The reaction mixture was concentrated and extracted with ethylacetate / water. The combined organic layers were dried over sodium sulphate and the solvent was evaporated. The title compound was obtained in 64 % yield after flash chromatography. **¹H-NMR (CDCl₃):** 7.97 d (*J* = 2.3 Hz, 1 H); 7.59 dd (J = 2.3 Hz / 8.7 Hz, 1 H); 7.47 dd (J = 7.5 Hz / 1.7 Hz, 1 H); 7.30 m (1 H); 6.96 m (3H); 4.62 m (1 H); 3.91 s (3H); 3.88 s (3H); 1.38 d (J = 8.7 Hz, 6H).
5c) 2-Isopropoxy-5-(2-methoxy-phenylethynyl)-benzoic acid
   A solution of 2-Isopropoxy-5-(2-methoxy-phenylethynyl)-benzoic acid methyl ester (280 mg) in methanolic KOH solution (10%) was stirred at ambient temperature overnight.The solvent was distilled off and the remaining solid was dissolved in water and extracted with ethylacetate. The water phase was acidified by addition of HCI (2 molar) and the water phase was extracted with ethylacetate (3x 50 ml). The combined organic layers were dried over sodium sulphate and evaporated to dryness. The title compound was obtained in 90 % yield. **¹H-NMR (DMSO-d₆):** 8.45 d (*J* = 2.3 Hz, 1 H); 7.75 dd (*J* = 2.3 Hz / 8.7 Hz, 1 H); 7.52 dd (*J* = 1.7 Hz / 7.5 Hz, 1 H); 7.37 m (1 H); 7.08 d (J = 8.7 Hz, 1 H); 6.97 m (2H); 4.94 m (1 H); 3.96 s (3H); 1.55 d (J = 6.0 Hz, 6H).
5d) N-[(R)-3-Hydroxy-1-(1H-indol-3-ylmethyl)-propyl]-2-isopropoxy-5-(2-methoxy-phenylethynyl)-benzamide
   A solution of 2-Isopropoxy-5-(2-methoxy-phenylethynyl)-benzoic acid (200 mg), (R)-3-Amino-4-(1H-indol-3-yl)-butan-1-ol (180 mg), HOBt monohydrate (131 mg) and EDC (164 mg) in DMF (10 ml) was stirred overnight at ambient temperature. The reaction mixture was poured into water and the resulting precipitate was filtered off. After drying the title compound was obtained in 65 % yield. **(DMSO-*d₆*):** 10.80 s (1H); 8.08 d (*J* = 8.5 Hz, 1 H); 7.78 d (*J* = 2.5 Hz, 1 H); 7.55 d (*J* = 7.9 Hz, 1 H); 7.53 dd (*J* = 8.7 Hz / 2.3 Hz, 1 H); 7.43 dd (*J* = 7.5 Hz / 1.5 Hz, 1 H); 7.35 m (1 H); 7.30 d (*J* = 7.9 Hz, 1 H); 7.14-6.88 m (6H); 4.71 m (1 H); 4.40 m (1 H); 4.32 s (1 H); 3.83 s (3H); 3.47 m (2H); 2.95 m (1 H); 2.90 m (1 H); 1.72 m (1 H); 1.58 m (1 H); 1.17 m (6H).

### Example 6

### 4-Ethoxy-3'-methoxy-biphenyl-3-carboxylic acid ((R)-3-hydroxy-1-naphthalen-1-ylmethyl-propyl)-amide

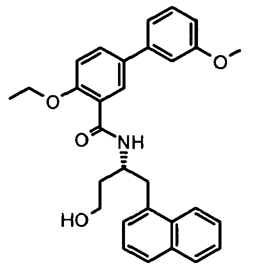

6a) 4-Ethoxy-3'-methoxy-biphenyl-3-carboxylic acid was prepared in analogy to WO 2007/017289, example 39a and 39b.
6b) 0.2 mmol Wang resin (0.6 mmol/g resin loading) is swollen for 15 min in 2 mL NMP. After filtration, the resin is reacted for 12h with 6 eq (R)-3-(9H-Fluoren-9-ylmethoxy-carbonylamino)-4-naphthalen-1-yl-butyric acid (0.3M in NMP), 6 eq pyridine and 6 eq 2,4-Dichlorobenzoylchloride under stirring. The resin in washed with NMP (3 x 2 mL) and capped for 5 minutes with 2 mL 10% acetanhydride (v/v) in DMF. After washing with DMF (3 x 2 mL), the Fmoc group is removed by treatment with 2 x 2 mL 20% piperidine in DMF (2 x 15 min). After washing with DMF, the 4-Ethoxy-3'-methoxy-biphenyl-3-carboxylic acid (2 eq, 0.3 M in NMP) is coupled with 2 eq HATU (0.3 M in NMP) and N-Methylmorpholine (4eq, 3M in NMP) for 4h at room temperature to the released amine moiety. After washing with NMP (3 x 2 mL) and THF (3 x 2mL), the alcohols are released from the resin by reduction with 2 mL 1M DIBAH in THF at 0°C. After 12 h, the resin is filtered and washed with THF (1 x 2mL). 1 mL 1 M KHSO₄ is added to the filtrate at room temperature, and the mixtures is evaporated to dryness. The residue is suspended in DMSO, filtered (2 x 2mL), and the filtrate is subjected to preparative HPLC.
   HPLC purification: Machine: Analytical 4 channel MUX system with CTC Pal injector, Waters 1525 Pumps, Waters 2488 UV detector and Waters ZQ 2000 single quad MS detector. Column X-Bridge RP C18 4.6 x 50 3.5µM; detection wavelength 214 nm; flow rate 1 ml/min; eluents A: 0.1% TFA in H₂O, B 0.1% TFA in ACN; gradient in each case based on B: 1% to 99% (10') to 99% (2') to 1% (0. 5') to 1% (2.5').
   Molecular peak (ESI, M+1): 471. Retention time: 10.6 min.
   The following compounds were obtained in analogy to the preparation methods described in detail:

| **Ex.** | **Product; reagents** | **Method analogous to** | **HPLC-MS** | **Structure** |
|---|---|---|---|---|
| **7** | 6-Methoxy-2-(3,4,5-trimethoxy-phenyl)-quinoline-4-carboxylic acid ((R)-3-hydroxy-1-naphthalen-1-yl-methyl-propyl)-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1 % to 99% (10') to 99% (2') to 1 % (0. 5') to 1% (2.5'). | 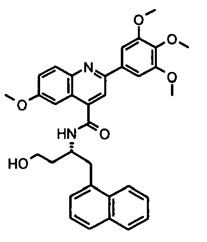 |
| | | | | |
| | *6-Methoxy-2-(3, 4, 5-trimethoxy-phenyl)-quinoline-4-carboxylic acid* | | | |
| | | | | |
| | *and* | | | |
| | | | | |
| | *(R)-3-(9H-Fluoren-9-yl-methoxycarbonylamino)-4-naphthalen-1-yl-butyric acid* | | Molecular peak (ESI, M+1): 568 | |
| | | | Retention time: 8.72 min. | |
| **8** | 6-Methoxy-2-(3,4,5-trimethoxy-phenyl)-quinoline-4-carboxylic acid ((R)-3-hydroxy-1-naphthalen-2-ylmethyl-propyl)-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1 % to 99% (10') to 99% (2') to 1% (0. 5') to 1% (2.5'). | 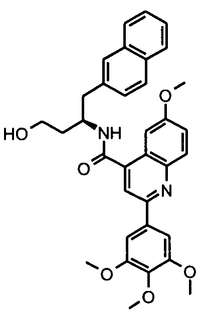 |
| | | | | |
| | *6-Methoxy-2-(3, 4, 5-trimethoxy-phenyl)-quinoline-4-carboxylic acid* | | | |
| | | | | |
| | *and* | | | |
| | | | | |
| | *(R)-3-(9H-Fluoren-9-ylmethoxycarbonylamino)-4-naphthalen-2-yl-butyric acid* | | Molecular peak (ESI, M+1): 568 | |
| | | | Retention time: 8.6 min. | |
| **9** | 6-Methoxy-2-(3,4,5-trimethoxy-phenyl)-quinoline-4-carboxylic acid ((R)-1-benzo-[b]thiophen-3-ylmethyl-3-hydroxy-propyl)-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1 % to 99% (10') to 99% (2') to 1% (0. 5') to 1% (2.5'). | 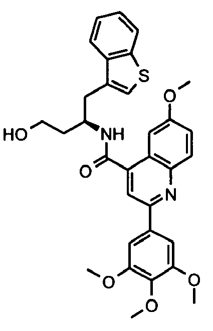 |
| | | | | |
| | *6-Methoxy-2-(3,4,5-trimethoxy-phenyl)-quinoline-4-carboxylic acid* | | | |
| | | | | |
| | *and* | | | |
| | | | | |
| | *(R)-4-Benzo[b]thiophen-3-yl-3-(9H-fluoren-9-ylmethoxy-carbonylamino)-butyric acid* | | Molecular peak (ESI, M+1): 574 | |
| | | | Retention time: 8.26 min. | |
| **10** | 6-Methoxy-2-(3,4,5-trimethoxy-phenyl)-quinoline-4-carboxylic acid ((R)-3-hydroxy-1-pyridin-3-ylmethylpropyl)-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1% to 99% (10') to 99% (2') to 1% (0. 5') to 1% (2.5'). | 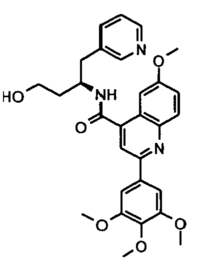 |
| | | | | |
| | *6-Methoxy-2-(3, 4, 5-trimethoxy-phenyl)-quinoline-4-carboxylic acid* | | | |
| | | | | |
| | *and* | | | |
| | | | | |
| | *(R)-3-(9H-Fluoren-9-yl-methoxycarbonylamino)-4-pyridin-3-yl-butyric acid* | | Molecular peak (ESI, M+1): 519 | |
| | | | Retention time: 5.51 min. | |
| **11** | 6-Methoxy-2-(3,4,5-trimethoxy-phenyl)-quinoline-4-carboxylic acid ((R)-3-hydroxy-1-thiophen-2-yl-methyl-propyl)-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1 % to 99% (10') to 99% (2') to 1% (0. 5') to 1% (2.5'). | 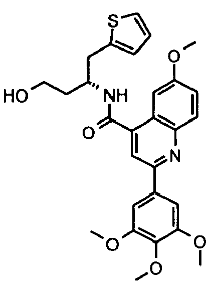 |
| | | | | |
| | *6-Methoxy-2-(3,4,5-trimethoxy-phenyl)-quinoline-4-carboxylic acid* | | | |
| | | | | |
| | *and* | | | |
| | | | | |
| | *(R)-3-(9H-Fluoren-9-yl-methoxycarbonylamino)-4-thiophen-2-yl-butyric acid* | | Molecular peak (ESI, M+1): 524 | |
| | | | Retention time: 7.52 min. | |
| **12** | 6-Methoxy-2-(3,4,5-trimethoxy-phenyl)-quinoline-4-carboxylic acid ((R)-3-hydroxy-1-thiophen-3-yl-methyl-propyl)-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1 % to 99% (10') to 99% (2') to 1% (0. 5') to 1% (2.5'). | 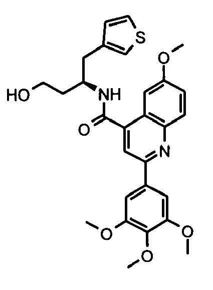 |
| | | | | |
| | *6-Methoxy-2-(3,4,5-trimethoxy-phenyl)-quinoline-4-carboxylic acid* | | | |
| | | | | |
| | *and* | | | |
| | | | | |
| | *(R)-3-(9H-Fluoren-9-yl-methoxycarbonylamino)-4-thiophen-3-yl-butyric acid* | | Molecular peak (ESI, M+1): 524 | |
| | | | Retention time: 7.47 min. | |
| **13** | 6-Methoxy-2-(3,4,5-trimethoxy-phenyl)-quinoline-4-carboxylic acid ((S)-1-furan-2-ylmethyl-3-hydroxy-propyl)-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1 % to 99% (10') to 99% (2') to 1% (0. 5') to 1% (2.5'). | 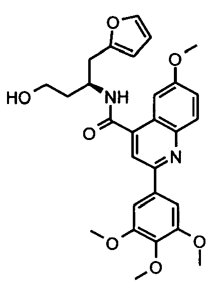 |
| | | | | |
| | *6-Methoxy-2-(3, 4, 5-trimethoxy-phenyl)-quinoline-4-carboxylic acid* | | | |
| | | | | |
| | *and* | | | |
| | | | | |
| | *(R)-3-(9H-Fluoren-9-yl-methoxycarbonylamino)-4-furan-2-yl-butyric acid* | | Molecular peak (ESI, M+1): 508 | |
| | | | Retention time: 7.57 min. | |
| **14** | 6-Methoxy-2-(3,4,5-trimethoxy-phenyl)-quinoline-4-carboxylic acid [(S)-3-hydroxy-1-(1H-indol-3-yl-methyl)-propyl]-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1% to 99% (10') to 99% (2') to 1 % (0. 5') to 1% (2.5'). | 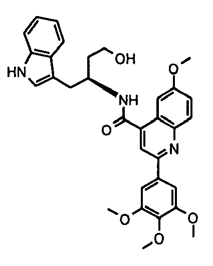 |
| | | | | |
| | *6-Methoxy-2-(3,4,5-trimethoxy-phenyl)-quinoline-4-carboxylic acid* | | | |
| | | | | |
| | *and* | | | |
| | | | | |
| | *(S)-3-(9H-Fluoren-9-yl-methoxycarbonylamino)-4-(1H-indol-3-yl)-butyric acid* | | Molecular peak (ESI, M+1): 557 | |
| | | | Retention time: 7.69 min. | |
| **15** | 6-Methoxy-2-(3,4,5-trimethoxy-phenyl)-quinoline-4-carboxylic acid ((R)-1-benzyl-3-hydroxy-propyl)-amide; | **6** | HPLC purification: Column X-Bridge RP C18 4.6 x 50 3.5µM; detection wave-length 214 nm; flow rate 1 ml/min; eluents A: 0.1 % TFA in H₂O, B 0.1 % TFA in ACN; gradient in each case based on B: 1 % to 99% (10') to 99% (2') to 1 % (0. 5') to 1% (2.5'). | 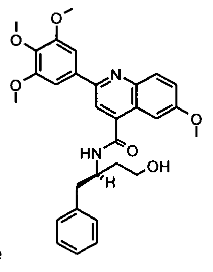 |
| | | | | |
| | *6-Methoxy-2-(3, 4, 5-trimethoxy-phenyl)-quinoline-4-carboxylic acid* | | | |
| | | | | |
| | *and* | | | |
| | | | | |
| | *(R)-3-(9H-Fluoren-9-yl-methoxycarbonylamino)-4-phenyl-butyric acid* | | Molecular peak (ESI, M+1): 518 | |
| | | | Retention time: 7.94 min. | |
| **16** | 4-Ethoxy-3'-methoxy-biphenyl-3-carboxylic acid ((R)-1-benzyl-3-hydroxypropyl)-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1% to 99% (10') to 99% (2') to 1% (0. 5') to 1 % (2.5'). | 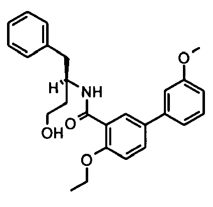 |
| | | | | |
| | *4-Ethoxy-3'-methoxy-bi-phenyl-3-carboxylic acid* | | | |
| | | | | |
| | *and* | | | |
| | | | | |
| | *(R)-3-(9H-Fluoren-9-yl-methoxycarbonylamino)-4-phenyl-butyric acid* | | Molecular peak (ESI, M+1): 421 | |
| | | | Retention time: 9.75 min. | |
| **17** | 6-Methoxy-2-(3,4,5-trimethoxy-phenyl)-quinoline-4-carboxylic acid ((S)-1-benzyl-3-hydroxy-propyl)-amide; | **6** | HPLC purification: Column X-Bridge RP C18 4.6 x 50 3.5µM; detection wavelength 214 nm; flow rate 1 ml/min; eluents A: 0.1 % TFA in H₂O, B 0.1 % TFA in ACN; gradient in each case based on B: 1 % to 99% (10') to 99% (2') to 1% (0. 5') to 1% (2.5'). | 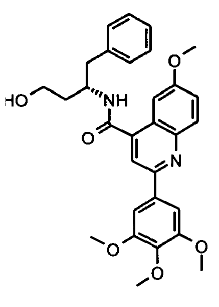 |
| | *6-Methoxy-2-(3,4,5-trimethoxy-phenyl)-quinoline-4-carboxylic acid* | | | |
| | | | | |
| | *and* | | | |
| | | | | |
| | *(S)-3-(9H-Fluoren-9-yl-methoxycarbonylamino)-4-phenyl-butyric acid* | | Molecular peak (ESI, M+1): 518 | |
| | | | Retention time: 7.57 min. | |

## Claims

1. Compounds of the formula I 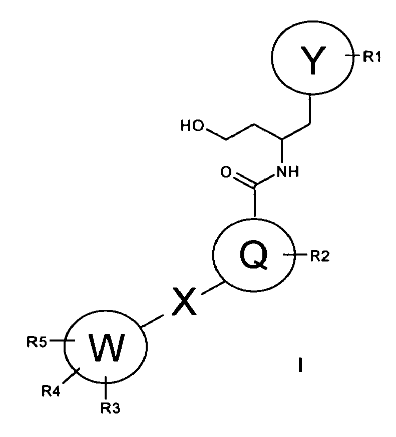 in which
R1 is hydrogen, halogen, cyano, C₁-C₆-alkyl;
R2 is hydrogen, halogen, cyano, C₁-C₆-alkyl, C₂-C₆-alkenyl or C₂-C₆-alkynyl, C₃-C₇-cycloalkyl, C₃-C₇-heterocycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene
where the hydrocarbon chains therein may optionally be substituted one or more times by fluorine or cyano;
R3, R4, R5 are independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl,
where the hydrocarbon chains therein may optionally be substituted one or more times by fluorine, cyano or the radicals: 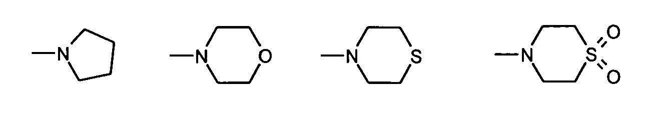 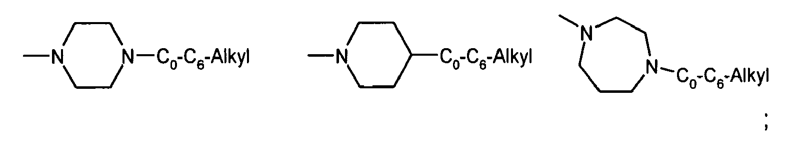 or
R3, R4, R5 are independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylam ino-C₁-C₆-alkylene, di(C₁-C₆)-alkylamino-C₁-C₆-alkylene, C₃-C₇-cydoalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneamino-carbonyl,
C₁-C₆-alkylcarbonyl, C₃-C₇-cycloalkylcarbonyl, carboxy, carboxamido [-C(O)NH₂], C₁-C₆-alkyloxycarbonyl,
C₁-C₃-alkylsulphanyl, C₁-C₆-alkysulphonyl, C₃-C₇-cycloalkylsulphonyl,
C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido,
-N(C₀-C₆-alkyl)-C(O)-C₁-C₆-alkyl, -N(C₀-C₆-alkyl)-C(O)-C₃-C₇-cycloalkyl,
-N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇cycloalkyl,
-N(C₀-C₆-alkyl)-SO₂-C₁-C₆-alkyl, -N(C₀-C₆-alkyl)-SO₂-C₃-C₇-cycloalkyl,
-N(C₀-C₆-alkyl)-SO₂-N-di(C₀-C₆-alkyl), -N(C₀-C₆-alkyl)-SO₂-NH-(c₃-C₇)-cycloalkyl,
-C(O)-N(H)-C₂-C₆-alkylene-(C₁-C₆-alkyl)amine,-C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the radicals: 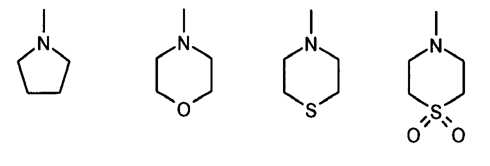 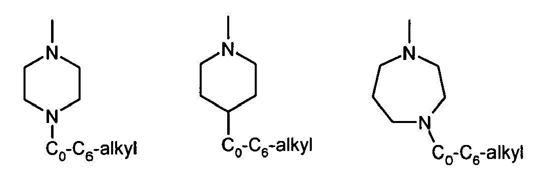 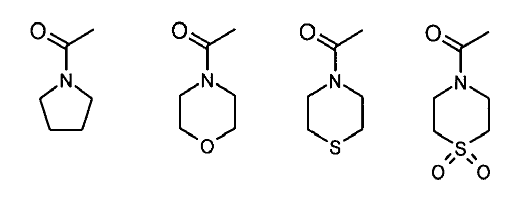 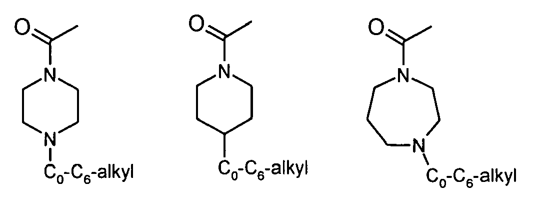 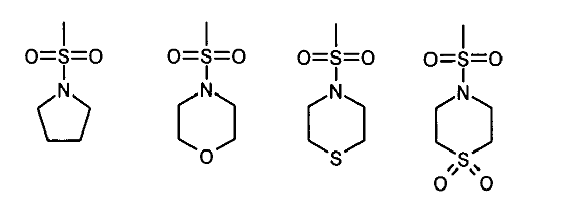 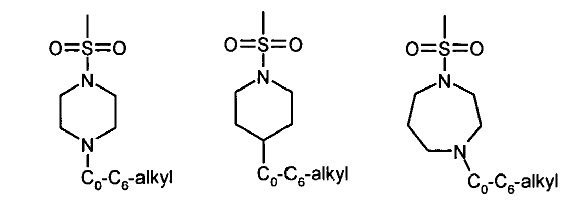 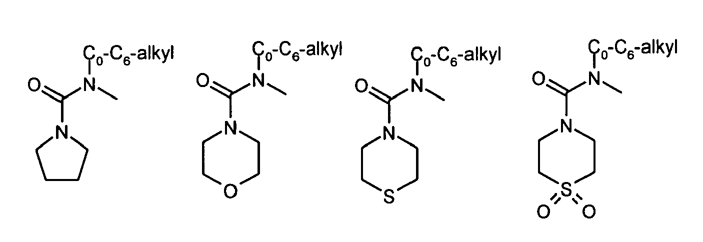 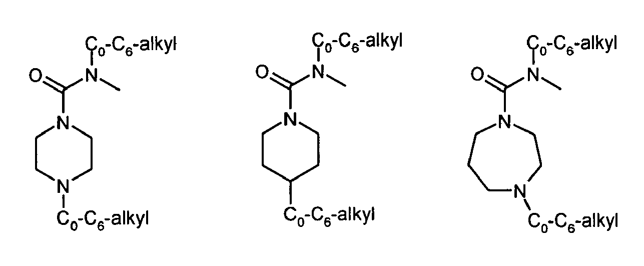 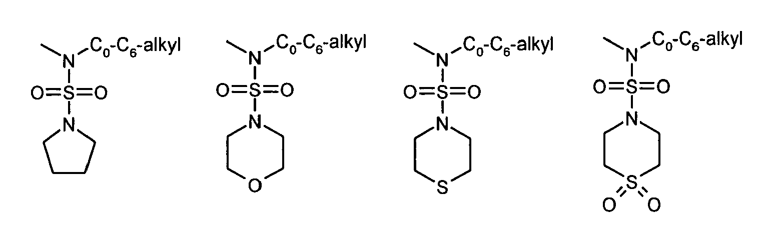 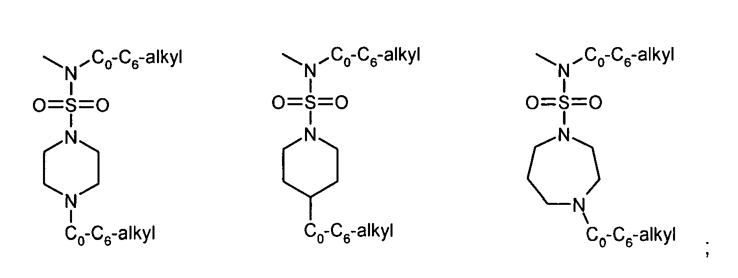
R3 and R4 may together form heterocycloalkyl, cycloalkyl;
Q is an aryl or heteroaryl group
or the biradical group 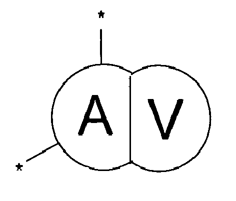 in which
A is a monocyclic aryl or a monocyclic heteroaryl group;
V is a cycloalkylen, cycloalkenylen, heterocycloalkylen or heterocycloalkenylen group;
X is a bond or ethynylene;
W is an aryl or heteroaryl group;
Y is an aryl or heteroaryl group;
where
R1 substitutes one or more positions of the aryl or heteroaryl ring in the radical Y;
R2 substitutes one or more positions of the aryl or heteroaryl ring
in the radical V, or
R2 substitutes one or more positions of the aryl or heteroaryl ring in the radical Q
with the provision that
if Q is a phenyl or pyridyl group
then
1) the R2 substituent in the para-position relative to the amide group may only be hydrogen; and
2) the group 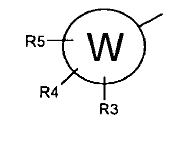
may not be a benzamide, substituted benzamide, phenylethanon, substituted phenylethanon, furan or thiophene.

2. Compounds according to claim 1, namely N-acyl homotryptophanols of the formula II 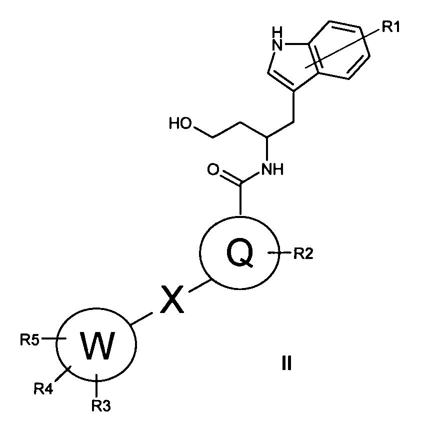 in which
R1, R2, R3, R4, R5, X, Q and W have the same meaning as defined in claim 1.

3. Compounds according to claims 1 or 2, namely N-acyl homotryptophanols of the formula III 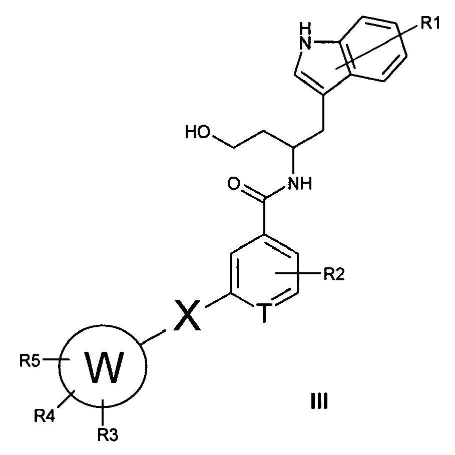 in which
T may be a nitrogen atom or a CH group; and
R1, R2, R3, R4, R5, X, and W have the same meaning as defined in claim 1.

4. Compounds according to claims 1 or 3, namely N-acyl homotryptophanols of the formula IV 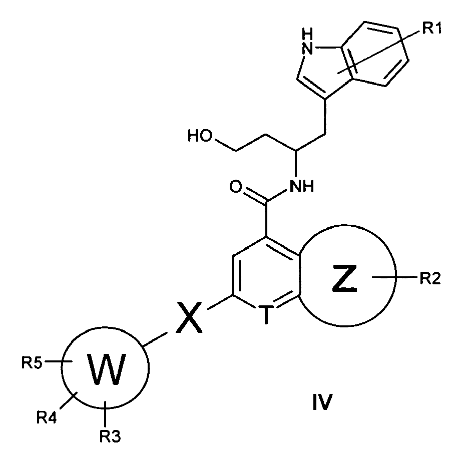 in which
T may be a nitrogen atom or a CH group;
Z is a cycloalkylen, cycloalkenylen, heterocycloalkylen or heterocycloalkenylen group or a a monocyclic aryl or a monocyclic heteroaryl group;
R1, R2, R3, R4, R5, X and W have the same meaning as defined in formula I,
where
R2 may substitute one or more positions of the radical Z.

5. Compounds according to any of the preceding claims, namely
| | |
|---|---|
| **1** | 6-Methoxy-2-(3,4,5-trimethoxy-phenyl)-quinoline-4-carboxylic acid [(R)-3-hydroxy-1-(1H-indol-3-ylmethyl)-propyl]-amide; |
| **2** | 4-Ethoxy-3'-methoxy-biphenyl-3-carboxylic acid [(R)-3-hydroxy-1-(1H-indol-3-ylmethyl)-propyl]-amide; |
| **3** | 3'-Chloro-4-isopropoxy-biphenyl-3,4'-dicarboxylic acid 3-{[(R)-3-hydroxy-1-(1H-indol-3-ylmethyl)-propyl]-amide} 4'-methylamide; |
| **4** | 4-Isopropoxy-3',4'-dimethoxy-biphenyl-3-carboxylic acid [(R)-3-hydroxy-1-(1H-indol-3-ylmethyl)-propyl]-amide; |
| **5** | N-[(R)-3-Hydroxy-1-(1H-indol-3-ylmethyl)-propyl]-2-isopropoxy-5-(2-methoxy-phenylethynyl)-benzamide; |
| **6** | 4-Ethoxy-3'-methoxy-biphenyl-3-carboxylic acid ((R)-3-hydroxy-1-naphthalen-1-ylmethyl-propyl)-amide; |
| **7** | 6-Methoxy-2-(3,4,5-trimethoxy-phenyl)-quinoline-4-carboxylic acid ((R)-3-hydroxy-1-naphthalen-1-ylmethyl-propyl)-amide; |
| **8** | 6-Methoxy-2-(3,4,5-trimethoxy-phenyl)-quinoline-4-carboxylic acid ((R)-3-hydroxy-1-naphthalen-2-ylmethyl-propyl)-amide; |
| **9** | 6-Methoxy-2-(3,4,5-trimethoxy-phenyl)-quinoline-4-carboxylic acid ((R)-1-benzo[b]thiophen-3-ylmethyl-3-hydroxy-propyl)-amide; |
| **10** | 6-Methoxy-2-(3,4,5-trimethoxy-phenyl)-quinoline-4-carboxylic acid ((R)-3-hydroxy-1-pyridin-3-ylmethyl-propyl)-amide; |
| **11** | 6-Methoxy-2-(3,4,5-trimethoxy-phenyl)-quinoline-4-carboxylic acid ((R)-3-hydroxy-1-thiophen-2-ylmethyl-propyl)-amide; |
| **12** | 6-Methoxy-2-(3,4,5-trimethoxy-phenyl)-quinoline-4-carboxylic acid ((R)-3-hydroxy-1-thiophen-3-ylmethyl-propyl)-amide; |
| **13** | 6-Methoxy-2-(3,4,5-trimethoxy-phenyl)-quinoline-4-carboxylic acid ((S)-1-furan-2-ylmethyl-3-hydroxy-propyl)-amide; |
| **14** | 6-Methoxy-2-(3,4,5-trimethoxy-phenyl)-quinoline-4-carboxylic acid [(S)-3-hydroxy-1-(1H-indol-3-ylmethyl)-propyl]-amide; |
| **15** | 6-Methoxy-2-(3,4,5-trimethoxy-phenyl)-quinoline-4-carboxylic acid ((R)-1-benzyl-3-hydroxy-propyl)-amide; |
| **16** | 4-Ethoxy-3'-methoxy-biphenyl-3-carboxylic acid ((R)-1-benzyl-3-hydroxy-propyl)-amide; |
| **17** | 6-Methoxy-2-(3,4,5-trimethoxy-phenyl)-quinoline-4-carboxylic acid ((S)-1-benzyl-3-hydroxy-propyl)-amide. |

6. Process for preparing compounds of the formula I of claim 1, wherein an aminoalcohol derivative V 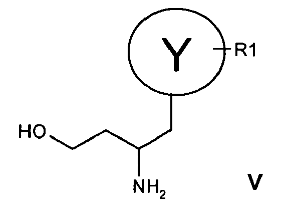 in which the radical R1 has the same meaning as defined in claim 1,
is coupled with a carboxylic acid of the formula VI 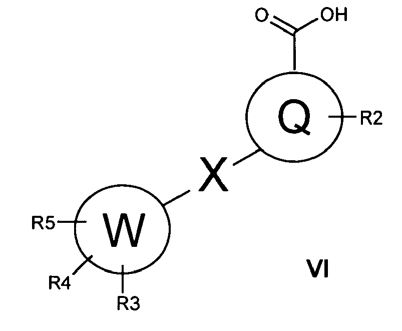 in which R2, R3, R4, R5, Q, X and W have the same meaning as defined in claim 1,
in an amide forming reaction comprising
a) conversion of said carboxylic acid into an intermediate active ester or carbonyl chloride with a suitable peptide-coupling reagent, or with thionyl chloride, oxalyl chloride, phosgene or derivatives thereof, where appropriate in the presence of a base,
b) reacting the active intermediate resulting from step a) with said aminoalcohol.

7. Process according to claim 6 for preparing compounds of the formula II of claim 2, wherein an aminoalcohol derivative of the formula VII 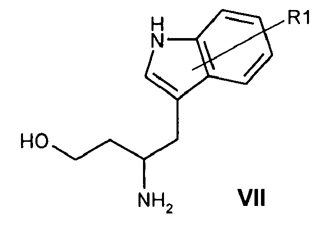 in which
R1 has the same meaning as defined in claim 1,
is coupled with the carboxylic acid of the formula VI.

8. Process according to claim 6 for preparing compounds of the formula III of claim 3, wherein the aminoalcohol derivative of the formula VII is coupled with a carboxylic acid of the formula VIII 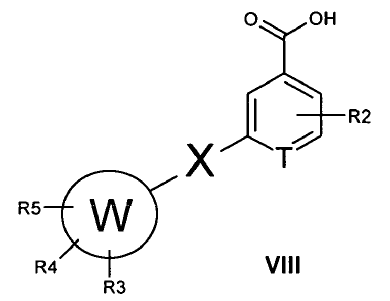 in which R2, R3, R4, R5, T, X, Y and W have the same meaning as defined in claim 3.

9. Process according to claim 6 for preparing compounds of the formula IV of claim 4, wherein the aminolacohol derivative of the formula VII is coupled with a carboxylic acid of the formula lX 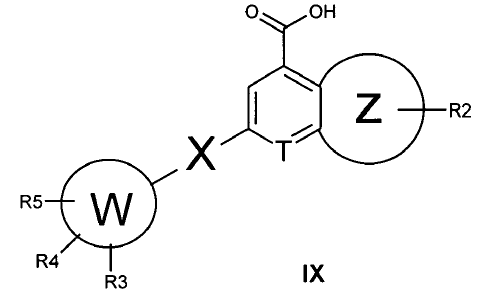 in which R2, R3, R4, R5, T, X and W have the same meaning as defined in claim 4.

10. Pharmaceutical compositions comprising at least one of the compounds according to any of claims 1 to 5 with pharmaceutically suitable excipients and/or carriers.

11. Use of the compounds of the general formula I according to any of claims 1 to 5 for the fertility regulation in men or in women.
